# EUROPEAN PATENT APPLICATION

(11) **EP 4 809 957 A1**
(43) Date of publication of application: **23.09.2026**
(21) Application number: 26165387.7
(22) Date of filing: 17.03.2026
(51) Int. Cl.: A61B 34/10, A61F 2/30, A61F 2/44

(54) **SYSTEMS FOR AUTOMATED FORMATION OF CUSTOMIZED IMPLANTS**

(30) Priority: 17.03.2025 US 202563773278 P
(71) Applicant: Blue Bend Medical Technologies Ltd., Toronto Ontario M2H 3N3 (CA)
(72) Inventor: KARMI, Davoud, Thornhill, ON L3T (CA); LEWIS, Stephen, North York, ON M5M 1K2 (CA); NIELSON, Christopher, York, ON M6S 2G2 (CA)
(74) Representative: J A Kemp LLP

(57) **Abstract**

Disclosed herein are systems and methods for automatically forming a cylindrical metal implant used for orthopedic surgery. A control system receives patient musculoskeletal data sets, derives spinopelvic alignment parameters, simulates candidate realignment configurations with estimated musculoskeletal forces, and generates an implant fabrication instruction set. An implant fabrication device comprising a bending assembly, a feeding assembly, and a controller circuit uses a three-point bending mechanism to locally bend successive discrete segments of a metal rod and globally deform the rod according to a desired postoperative spinal alignment. The feeding system intermittently advances the rod into the bending mechanism and rotates to change the bending direction. Force and angle sensors provide feedback to accurately deform the rod to the specified angle taking into account elastic spring-back. An intraoperative verification module compares post-attachment alignment against the target and triggers re-bending if needed.

## Description

### CROSS-REFERENCE

This application is a non-provisional of, and claims all benefit, including priority to, US Application No. 63/773,278 filed on 03/17/2025 and entitled SYSTEMS AND METHODS FOR AUTOMATED FORMATION OF CUSTOMIZED IMPLANTS, incorporated herein by reference in its entirety.

### FIELD

Embodiments of the present disclosure relate to the field of medical and surgical instrumentation and associated manufacturing devices and computerized control instructions execution for precision automated implant fabrication, and more specifically, embodiments relate to devices, systems and methods for improved mechanical-deformation-based production of metallic implants based on a desired orthopedic alignment.

### INTRODUCTION

The precise manufacturing of surgical implants for orthopedic surgery is useful as the implants stabilize the affected musculoskeletal tissues and promote proper healing. Orthopedic surgery, in particular, spinal surgery, can occur as a result of spinal deformities that can either be trauma related or physiological. Example spinal deformities include scoliosis, stenosis, lordosis. Specifically, in spinal fusion surgeries, dimensions of the rod implants maintain the correct alignment of the vertebrae, which is used as part of the fusion process. Deviations in the manufacturing process can lead to complications such as implant failure, misalignment, or even damage to surrounding tissues. An insufficient level of manufacturing precision ultimately jeopardizes surgical outcomes, prolongs recovery times, and diminishes quality of life for patients undergoing surgery. It is important to note that precision manufacturing of surgical implants is a difficult technical endeavor as there is an inability to easily correct fabrication errors, and there are real world and practical nonidealities in the manufacturing process.

Moreover, the diverse musculoskeletal structures of patients present significant challenges in the manufacturing of implants for orthopedic surgery. Each patient's anatomy varies in terms of bone density, curvature, and overall alignment, requiring implants to be highly customizable; and the observable spinal characteristics pre-op may not match intra-op observations. This variability demands advanced measurement, simulation, planning, and manufacturing techniques to produce and potentially refine implants that can precisely match the unique contours and biomechanical needs of individual patients. While it is possible for the implant to be prefabricated based on a target alignment plan, it is more desirable to have the implant fabricated in-situ, for example, during or as part of a medical procedure. Dynamically generating and updating instruction sets as well as being able to fabricate on the spot allows for in-process adjustments that would otherwise not be possible.

There is therefore a need for improvements in precision manufacturing of rod implants according to the patient's specific musculoskeletal structures and desired alignment profiles for orthopedic surgeries.

### SUMMARY

Spinal fusion surgery uses precision contouring of cylindrical metal rod implants so that, once affixed to pedicle screws along a plurality of vertebral levels, the rod implant entrains the spine into a desired postoperative alignment. Entrainment is difficult because in the operating theater there is little time and room for mistakes. However, entrainment is subject to considerations of physical phenomena that can only be identified as part of the fabrication process as well as the modified physical constraints of a person's physical anatomy which may change during surgical procedures. Finally, there can be differing physical characteristics of different geometries, thickness, material selection of the entrainment device, which impact physical features such as spring-back, compressive, tensile, and shear forces.

Clinical workflows that rely on manual rod bending performed by a surgeon in the operating theatre is subjective, non-repeatable, and unable to account quantitatively for patient-specific biomechanical forces, elastic spring-back of the implant material, or the mechanical stiffness of the patient's own spinal column after intraoperative osteotomy and release procedures. These technical deficiencies propagate into postoperative malalignment, which leads to mechanical revision surgery and complications including proximal junctional failure, proximal junctional kyphosis, rod fracture, sacral fracture, adjacent segment disease, and pseudoarthrosis. There is accordingly a need for an integrated computer-controlled system that interfaces between patient-specific surgical planning and precision implant fabrication. The computer-integrated control system controls a physical implant fabrication device that, dynamically, and during a procedure, can be used to precisely fabricate a physical implantation device, which can be a rod.

Practical integration of this control system can further include a physical stiffness measuring device, which can be a physical probe that is used to apply a force and measure a reaction, which can include deformation and reactionary forces. The deformation can be temporary (i.e. elastic) and the physical probe can be employed after intermediate surgical procedures have been performed, such as spinal fusions, discectomies, or other resurfacing has occurred. In particular, spinal fusion can include the implantation of hardware and fastening devices such as screws, rods, plates, and cages, which when implanted earlier in the surgical procedure, could impact how the implant is fabricated at a later stage in the surgical procedure.

Disclosed herein is a precision implant fabrication process executed by a control system that receives patient musculoskeletal data sets, derives spinopelvic alignment parameters, simulates candidate spinal realignment configurations with estimated musculoskeletal forces, and based on a desired spinal realignment configuration, generates a machine-executable implant fabrication instruction set. Executing the instruction set, an implant fabrication device comprising a bending assembly, a feeding assembly, and a controller circuit, autonomously advances, orients, and deforms successive discrete segments of a metal surgical implant while compensating for elastic spring-back. A verification module of the implant fabrication system takes intra-operative measurements upon attachment of the implant to verify the resulting spinal alignment with the desired spinal realignment configuration and flags the implant for refabrication in case of significant deviation. By coupling biomechanical simulation with closed-loop fabrication control, the system provides a quantitative, repeatable, and patient-specific manufacturing pipeline that operates intraoperatively, thereby enabling real-time iterative correction of the implant even after the spine has been surgically modified.

The system further provides, through a user interface, a plurality of graphical and computational tools configured to derive spinopelvic alignment parameters from medical imaging, compare candidate realignment configurations against a plurality of alignment classification criteria, render estimated musculoskeletal forces at each vertebral level, and compute a risk factor of mechanical complication before any physical deformation of the orthopedic implant is commenced. The planning output is an implant deformation target data set that defines, for each discrete segment of the implant, a target deformation angle, a target deformation orientation, and a corresponding segment length, which together constitute a complete machine-readable fabrication programme. These technical features collectively transform the implant manufacturing process from an ad hoc manual procedure into an objective, data-driven, and computationally verifiable fabrication operation.

The user interface renders interactive graphical control elements, such as sliders, adjustment wheels, and input boxes which are then used to update or modify a simulation and ultimately be used to generate the implant fabrication instruction set. The graphical control elements can further include a graphical representation of a cone of economy as well as overlaid graphical representations of pre-operative and potential post-operative outcomes. The post-operative outcomes are limited by a combination of what the person's physiology can support as well as practical limitations of the entrainment hardware. The user interface is coupled to other modules such as the measurement module, simulation module, planning module, and verification module, as well as an intraoperative stiffness measuring device.

More specifically, a computer processor within a control system is configured to receive patient musculoskeletal data sets comprising spinal characteristic data objects. The spinal characteristic data objects are derived at least from medical imaging of a patient's spine, where the medical imaging may include X-ray imaging (including biplanar imaging such as EOS), computed tomography (CT), and magnetic resonance imaging (MRI). From these data objects, the processor is configured to derive a plurality of spinopelvic alignment parameters, which are quantitative descriptors of the spatial relationships between vertebral bodies, the sacrum, the pelvis, and the global sagittal balance of the patient. The spinopelvic alignment parameters may number in excess of fifty discrete parameters including but not limited to geometric angles and offsets between vertebrae and the pelvis in both the sagittal and coronal planes.

Based on the derived plurality of spinopelvic alignment parameters, the computer processor is configured to simulate one or more candidate realignment configurations based on different alignment classification criteria. Each candidate realignment configuration is a computationally generated data structure representing a set of segmental changes required across a plurality of vertebral levels to achieve a desired postoperative spinal alignment. The simulation involves applying, for a given upper instrumented vertebra (UIV) and lower instrumented vertebra (LIV) selection, a mathematical transformation to the baseline preoperative spinal geometry such that the resulting simulated spinal geometry satisfies a desired target alignment criterion. The processor further computes, for each candidate realignment configuration, estimated musculoskeletal forces at one or more vertebral levels, where the forces comprise at least shear forces and compression forces at each intervertebral level, determined by solving a patient-specific musculoskeletal model that accounts for the patient's sex, weight, height, and spinal curvature as input parameters to an inverse dynamics computation. The simulated alignment results may be rendered for display at a user interface for a medical professional in making medical decisions regarding a specific treatment plan. Alternatively, the control system automatically suggests an optimal treatment plan with a correspondingly designed implant rod profile.

Upon selection of a realignment configuration, the control system generates an implant fabrication instruction set comprising a deformation target data set. The deformation target data set defines, for each discrete segment of the orthopedic implant, three parameters: a target deformation angle, specifying the magnitude of plastic deformation to be applied at that segment; a target deformation orientation, specifying the rotational plane in which the bend is to be applied; and a corresponding segment length, specifying the linear distance along the longitudinal axis of the implant rod between successive bend points. These parameters are computed by a mathematical decomposition of the desired rod profile, which may employ freeform curve representations including but not limited to Bézier curves, into a finite sequence of discrete bending operations. The decomposition algorithm ensures that the aggregate of all discrete bends yields a smooth, continuous curve that conforms to the selected postoperative alignment profile.

The physical implant fabrication device comprises a bending assembly, a feeding assembly, and a controller circuit. The bending assembly can include the grippers and bellows that bend the implant in accordance with specific instruction sets. The bending assembly an also include sensors that measure the reactionary forces encountered by the bending assembly during bending of a segment. The feeding assembly can include a conveyor, rollers, and pushers / pullers that obtain rod lengths from a feedstock required for the specific fabrication instructions relating to the desired implant shape. The controller circuit is electronically coupled to the bending assembly and the feeding assembly by way of electrical connections that can provide a flow of electrical current or digitized instruction sets, that for example, are coupled to specific servo motors of the bending assembly. The controller circuit, for example, is a circuit board having onboard computing parts or microcontrollers that are configured to cause the implant fabrication device to advance, via the feeding assembly, successive discrete segments of the orthopedic implant into the bending assembly according to the corresponding segment length specified in the deformation target data set.

**In** an embodiment, the circuit board has onboard memory that includes data storage of previous bending segment's reaction forces measured during the bending process, which can be used as an input when controlling the bending of subsequent sections. This is important because physically proximate bending joints can impact the bending forces required to bend the next section or can have impacts on the overall mechanical properties of the output implant. Measuring these forces, especially in conjunction with the stiffness measuring device outputs, provides an advantage when dynamically fabricating implants in real time during a procedure.

The feeding assembly includes a linearly movable clamp that is intermittently activated to frictionally engage the cylindrical metal implant and translate it axially into the bending region by a precisely controlled linear displacement. The controller circuit is further configured to adjust, via the feeding assembly, a bending orientation of the orthopedic implant according to the target deformation orientation, which is accomplished by a rotator motor that alters the rotational orientation of the implant about its longitudinal axis so that subsequent bending operations can be applied on different planes.

The controller circuit is further configured to apply, via the bending assembly, a controlled deformation to the orthopedic implant according to the target deformation angle and a spring-back compensation magnitude based on elastic deformation properties (e.g., modulus) of the orthopedic implant. The spring-back compensation is effectuated by deforming the implant beyond the target deformation angle, holding the deformed position for a predetermined dwell period, retracting the bending assembly, measuring a resulting deformation angle after retraction, and iteratively repeating the deformation and retraction sequence until the measured deformation angle matches the target deformation angle within a predetermined tolerance. The magnitude of the overbending and the duration of the dwell time may be adjusted based on a past fabrication of an implant of comparable material and shape.

In some embodiments, the processor utilises a trained artificial intelligence model to predict postoperative alignment parameters of the unfused spinal segments. The artificial intelligence model receives as input the preoperative spinal geometry, the selected realignment configuration, and patient demographic data, and outputs predicted values for parameters such as sacral slope and T1 pelvic angle in the unfused spine. The predicted unfused spine alignment is used to refine the implant fabrication instruction set, as the final postoperative spinal geometry depends not only on the contour of the rod implant but also on the compensatory response of the uninstrumented vertebral segments above and below the fusion construct.

In some embodiments, the computer processor is further configured to receive intraoperative spinal alignment data obtained after attachment of the orthopedic implant to the patient's spine. The processor compares the intraoperative spinal alignment data against the desired postoperative spinal alignment and either verifies compliance or generates an updated implant fabrication instruction set for execution by the implant fabrication device. This closed-loop intraoperative verification enables the system to detect segment-by-segment deviations between the targeted and actual postoperative alignments, quantify the resulting musculoskeletal force differentials, and determine whether the rod implant should be removed, re-bent, and reattached before surgical closure.

The control system may further comprise a stiffness measurement subsystem configured to apply a predetermined displacement or force onto the patient's spine and measure a resulting force or displacement to derive a spine stiffness parameter. The stiffness measurement may be performed intraoperatively after osteotomy and release procedures have been carried out, at which point the mechanical properties of the spine differ from their preoperative state. The spine stiffness parameter is received by the computer processor, which modifies the implant fabrication instruction set by applying an overbend correction to the deformation target data set, such that when the orthopedic implant is attached and the spine exerts its resistive forces on the rod, the patient's spine assumes the desired postoperative alignment. In some embodiments, the stiffness measurement is performed by a robotic arm that functions as a three-point loading apparatus, applying a controlled force at a specific vertebral level and measuring the resulting displacement, from which the local segmental stiffness is computed as force divided by displacement.

The control system is further configured to modify the implant fabrication instruction set to minimise a risk factor of mechanical complication, the risk factor computed based on the estimated musculoskeletal forces at one or more vertebral levels in combination with a bone mineral density of the patient. The risk factor of mechanical complication comprises an estimated probability in the occurrence of one or more of proximal junctional failure, proximal junctional kyphosis, rod fracture, sacral fracture, adjacent segment disease, and pseudoarthrosis. The bone mineral density may be derived from CT imaging using phantomless calibration techniques and combined with the computed vertebral loading to assess whether the forces at a given vertebral level exceed a failure threshold for that patient's bone quality. In some embodiments, the processor further accounts for instrumental failure modes including rod fracture risk based on the rod material, rod dimensions, and the computed cyclic loading expected from the patient's daily motion activities.

The control system may be further configured to compare the one or more candidate realignment configurations against a plurality of alignment classification criteria comprising one or more of a Global Alignment and Proportion (GAP) classification, an age-adjusted alignment, a Roussouly classification, a segmental alignment, and a T4-L1 axis alignment. For each classification criterion, the processor generates a corresponding simulated postoperative spinal geometry and computes the associated musculoskeletal forces, thereby enabling a quantitative comparison across classification paradigms. The processor is further configured to render a cone of economy visualisation indicating musculoskeletal expenditure for each candidate realignment configuration, where the cone of economy represents a spatial envelope within which the patient's centre of gravity can be maintained with minimal muscular effort.

In some embodiments, the spinal characteristic data objects further comprise dynamic motion data sets from one or more inertial measurement units (IMUs) positioned on the patient's vertebrae. The dynamic motion data sets are representative of patient-specific dynamic alignment and capture the patient's spinal kinematics during functional activities such as sit-to-stand transitions, gait, and flexion-extension movements. The processor integrates the dynamic motion data into the musculoskeletal force computation such that the estimated vertebral loading reflects not only the static standing alignment but also the dynamic forces that the spine and the attached implant will experience during the patient's habitual movement patterns. In some embodiments, the placement of the IMUs may be dictated by the measurements from spinal mouse devices that identify the vertebral level prior to the IMU attachment for accurate placement of the sensor units.

The control system may be further configured to render a graphical display of the plurality of spinopelvic alignment parameters of a simulated baseline spine and the desired postoperative spinal alignment as colored bars, each colored bar indicating a degree of deviation from the desired postoperative spinal alignment. The processor is further configured to render, for display in real time as the baseline spine is modified toward the desired postoperative spinal alignment, segmental Cobb angles at each vertebral level in one or both of sagittal and coronal planes, wherein each vertebra is rendered with a color corresponding to a magnitude of difference between a baseline Cobb angle and a target Cobb angle. This color-coded rendering enables the graphical user interface to communicate, at each vertebral level, the type and magnitude of corrective procedure required, such as a posterior column osteotomy, a pedicle subtraction osteotomy, or an interbody cage insertion, without requiring the operator to perform manual calculations.

In an example use case directed to adult spinal deformity correction, biplanar radiographic images are captured of a patient presenting with degenerative lumbar scoliosis and sagittal imbalance. The computer processor receives the imaging data as patient musculoskeletal data sets, automatically extracts the spinal characteristic data objects, and derives a plurality of spinopelvic alignment parameters including pelvic incidence, sacral slope, lumbar lordosis, thoracic kyphosis, and sagittal vertical axis. The processor then simulates one or more candidate realignment configurations for a planned fusion from T10 to the sacrum, computing for each candidate realignment configuration the estimated musculoskeletal forces at each vertebral level from T12 to S1. The user interface renders each candidate realignment configuration overlaid on the baseline spinal geometry, with colored bars at each vertebral level indicating the segmental Cobb angle deviation from the desired postoperative spinal alignment. Upon selection of a candidate realignment configuration, the processor generates the implant fabrication instruction set comprising the deformation target data set, which defines for each discrete segment of the orthopedic implant the corresponding segment length, target deformation angle, and target deformation orientation. The implant fabrication device, situated in the operating theatre, receives the implant fabrication instruction set, and the controller circuit causes the implant fabrication device to advance, adjust, and apply controlled deformations to the orthopedic implant through a sequence of discrete segments, compensating for elastic spring-back via the spring-back compensation magnitude at each bend point, to produce a finished orthopedic implant conforming to the selected realignment configuration.

After the orthopedic implant is attached to pedicle screws along the instrumented vertebral levels, intraoperative imaging is then acquired, and the control system receives the resulting intraoperative spinal alignment data. The processor compares the intraoperative spinal alignment data against the desired postoperative spinal alignment on a segment-by-segment basis and renders the deviations as color-coded indicators on the user interface. Where the processor determines that the estimated musculoskeletal forces at a given vertebral level exceed a predetermined threshold or that the segmental Cobb angle deviation exceeds a predetermined tolerance, the processor generates an updated implant fabrication instruction set for execution by the implant fabrication device. The orthopedic implant is then removed, re-processed by the implant fabrication device according to the updated implant fabrication instruction set, and reattached, thereby enabling iterative intraoperative verification of compliance to the desired postoperative spinal alignment before surgical closure.

In a further example use case, the control system incorporates intraoperative spine stiffness measurement. After osteotomy and release procedures are performed, the stiffness measurement subsystem applies a predetermined displacement or force at one or more vertebral levels and measures the resulting force or displacement to derive a spine stiffness parameter. The control system receives the spine stiffness parameter, computes the segmental stiffness at each measured level, and modifies the implant fabrication instruction set by applying an overbend correction to the deformation target data set such that when the orthopedic implant is attached and the spine's resistive stiffness acts upon the orthopedic implant, the patient's spine assumes the desired postoperative alignment. The user interface displays the stiffness-corrected rod profile alongside the original target profile, and the implant fabrication device automatically processes the orthopedic implant according to the modified implant fabrication instruction set. The entire measurement, computation, and fabrication cycle is completed intraoperatively, enabling production of a patient-specific orthopedic implant that accounts for the actual mechanical state of the surgically modified spine rather than relying solely on preoperative imaging data.

Corresponding methods and computer program products, including non-transitory computer readable media storing machine-interpretable instruction sets that, when executed by a computer processor, cause the computer processor to perform the above-described operations, are contemplated.

### DESCRIPTION OF THE FIGURES

In the figures, embodiments are illustrated by way of example. It is to be expressly understood that the description and figures are only for the purpose of illustration and as an aid to understanding.

Embodiments will now be described, by way of example only, with reference to the attached figures, wherein in the figures:
**FIG. 1** is a block diagram of an example system for automated intraoperative formation of an orthopedic implant, showing the control system and its functional modules, according to some embodiments.
**FIG. 2** is a block diagram illustrating the system architecture and data flow among the measurement module, the simulation module, the planning module, the implant fabrication device, and the intraoperative verification module, according to some embodiments.
**FIG. 3** is a graphical representation of a patient's spine as rendered by the measurement module, illustrating the individual vertebral levels from the cervical through the lumbar spine, according to some embodiments.
**FIG. 4** is an example display of spinopelvic alignment parameters derived by the measurement module from patient musculoskeletal data sets, showing segmental Cobb angles in coronal, sagittal, and spatial views, according to some embodiments.
**FIG. 5** is a simulation display generated by the simulation module, showing a plurality of candidate realignment configurations generated according to a plurality of alignment classification criteria together with a tabular comparison of spinopelvic alignment parameters for each candidate realignment configuration, according to some embodiments.
**FIG. 6** is a simulation results display rendered by the simulation module, showing simulated postoperative spinal geometries for each of a plurality of candidate realignment configurations generated according to different alignment classification criteria, according to some embodiments.
**FIG. 7** is a sagittal simulation showing spinopelvic parameters and sagittal Cobb angles in the fused part with colored bars indicating a degree of deviation between a baseline spine and a target spine, together with per-level adjustment controls, according to some embodiments.
**FIG. 8** is a coronal simulation showing coronal Cobb angles in the fused part with colored bars indicating a degree of deviation between a baseline spine and a target spine, together with per-level adjustment controls, according to some embodiments.
**FIG. 9** shows example estimated musculoskeletal forces in compression, anteroposterior (AP) shear, and lateral shear at each vertebral level for preoperative and postoperative spinal alignments, according to some embodiments.
**FIG. 10** is a risk factor computation output showing the estimated musculoskeletal forces at the UIV and UIV+1 levels, together with aggregated risk factors of mechanical complication, according to some embodiments.
**FIG. 11** is an example rod profile output generated by the planning module, showing the rod length, curve length up to the inflection point, Cobb angles, interbody angles, and PCO angles for each vertebral level, according to some embodiments.
**FIG. 12** is a system schematic diagram showing the integration of the control system, the user interface, and the implant fabrication device, including the actuation subsystem, force measurement system, angle measurement system, and feeding system, according to some embodiments.
**FIG. 13** is an isometric view of the implant fabrication device, showing the servo motor, coupling, saddle, ball screw, guides, force magnifier mechanism, moving rollers, fixed roller, and cylindrical metal implant, according to some embodiments.
**FIG. 14A** is a close-up isometric view of the feeding assembly, showing the electro-magnetic solenoid, stepper motors, wedge components, V-shaped grippers, and associated drive mechanisms, according to some embodiments; **FIG. 14B** is a cross-section of the feeding assembly of **FIG. 14A**, according to some embodiments.
**FIG. 15A** is a close-up isometric view of an alternative embodiment of the feeding assembly employing a disposable sterilised cartridge, according to some embodiments; **FIG. 15B** is a cross-section of the feeding assembly of **FIG. 15A****,** according to some embodiments.
**FIG. 16A** is a close-up isometric view of the gripper elements of the alternative feeding assembly of **FIG. 15A**, showing the four grippers and their hinge arrangement, according to some embodiments.
**FIG. 16B** illustrates an isometric view of an alternative embodiment of the gripper assembly in a disassembled configuration, according to some embodiments.
**FIG. 16C** illustrates a detail view of an individual gripper jaw of the alternative gripper assembly, according to some embodiments.
**FIG. 16D** illustrates a detail view of the outer sleeve of the alternative gripper assembly, according to some embodiments.
**FIG. 16E** illustrates a cutaway view of the alternative gripper assembly in an assembled state, according to some embodiments.
**FIG. 17** is a flowchart illustrating the computational steps of a method for generating an implant fabrication instruction set, according to some embodiments.
**FIG. 18** is a flowchart illustrating the execution steps of the implant fabrication instruction set at the implant fabrication device, including advancing, adjusting, and applying controlled deformations, according to some embodiments.
**FIG. 19** is a block diagram of a computing device suitable for implementing the control system, according to some embodiments.

### DETAILED DESCRIPTION

In clinical practice, the contouring of rod implants for spinal fusion surgery is performed manually by the operating surgeon. This manual process is inherently subjective and non-repeatable: the surgeon must rely on visual estimation and tactile feedback to approximate the desired spinal curvature, without quantitative guidance as to the biomechanical forces that the implant will impose on the patient's vertebrae once attached. Manual rod bending also fails to account for the elastic spring-back properties of the implant material, which cause the rod to partially recover from each bend once the bending force is removed, resulting in an unpredictable final rod geometry. Furthermore, the surgeon has no means of quantitatively assessing, before or during the bending process, how the resulting rod profile will affect the distribution of musculoskeletal forces across the instrumented vertebral levels, or whether the forces at critical levels, such as the upper instrumented vertebra (UIV) and the vertebra immediately above it (UIV+1), will exceed thresholds associated with postoperative mechanical complications. The mechanical stiffness of the patient's spine, which changes intraoperatively as osteotomy and release procedures are performed, further confounds the manual bending process because the surgeon cannot predict the equilibrium geometry that will result when the rod is attached to pedicle screws and the spine's resistive forces act upon the implant. These technical deficiencies propagate into measurable postoperative malalignment and elevated complication rates, and there is accordingly a need for an integrated computer-controlled system that quantitatively links patient-specific biomechanical analysis with precision implant fabrication in a closed-loop intraoperative workflow.

Embodiments herein provide systems, methods, and devices for automated intraoperative formation of an orthopedic implant for attachment during spinal fusion surgery. An integrated computational pipeline receives patient musculoskeletal data sets, derives spinopelvic alignment parameters, simulates candidate spinal realignment configurations with estimated musculoskeletal forces, and generates a patient-specific implant fabrication instruction set. An implant fabrication device then autonomously fabricates the orthopedic implant in the operating theatre using sensor feedback, enabling real-time correction and iterative refinement of the implant profile while the patient remains in the operating theatre.

The orthopedic implant can be a cylindrical metal implant, such as a rod fabricated from cobalt-chromium (CoCr) or titanium alloy, having a diameter of, for example, 5.5 mm or 6.0 mm, although other dimensions and materials are contemplated. As used herein, the term "orthopedic implant" can refer to any elongated structure that is plastically deformable and is configured for fixation to vertebral anatomy, including but not limited to spinal fusion rods.

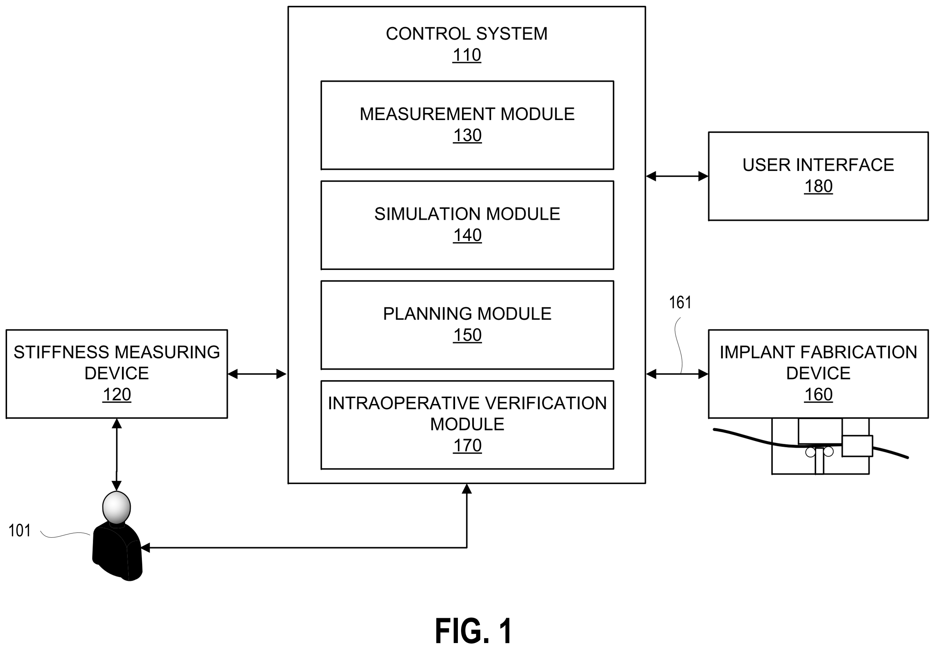

**FIG. 1** is a block diagram of an example system for automated intraoperative formation of the orthopedic implant. The system operates on a person 101 and comprises a control system 110, a stiffness measuring device 120, a user interface 180, and an implant fabrication device 160. The control system 110 further comprises a measurement module 130, a simulation module 140, a planning module 150, an intraoperative verification module 170. Each of the measurement module 130, the simulation module 140, the planning module 150, and the intraoperative verification module 170 may be implemented as a software module stored in the non-transitory computer-readable storage medium and executed by the computer processor, as a hardware module comprising dedicated processing circuitry, or as a combination thereof.

The control system 110 comprises a computer processor coupled with computer memory and a non-transitory computer-readable storage medium. The computer processor of the control system 110 is configured to execute the measurement module 130, the simulation module 140, the planning module 150, and the intraoperative verification module 170, and to communicate with the implant fabrication device 160 and the user interface 180. The implant fabrication device 160 is configured to execute a fabrication instruction set 161 produced by the control system 110. The control system 110 may be implemented as a dedicated computing device, or a distributed processing system comprising a plurality of communicatively coupled processors.

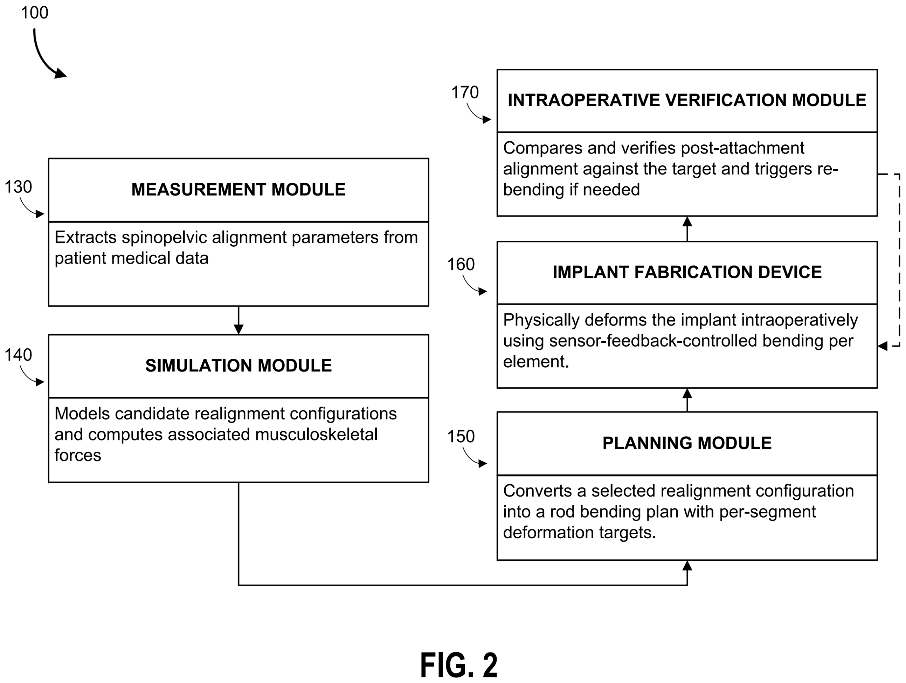

**FIG. 2** is a module interaction diagram showing the data flow among the functional modules of the system 100. The measurement module 130 receives the patient musculoskeletal data sets, extracts the spinopelvic alignment parameters, and forwards the spinopelvic alignment parameters to the simulation module 140. The simulation module 140 receives the spinopelvic alignment parameters, models the one or more candidate realignment configurations according to the plurality of alignment classification criteria, computes the associated estimated musculoskeletal forces at one or more vertebral levels for each candidate realignment configuration, computes the risk factor of mechanical complication for each candidate realignment configuration, and renders the cone of economy visualisation, force comparison displays, and risk factor computation outputs via the user interface 180. Upon operator selection of a candidate realignment configuration via the user interface 180, the simulation module 140 forwards the selected candidate realignment configuration and its corresponding simulated postoperative spinal geometry to the planning module 150. The planning module 150 receives the selected candidate realignment configuration, enables operator-driven corrective procedure specification via the user interface 180, converts the corrected realignment configuration into the implant fabrication instruction set 161 comprising the deformation target data set, and transmits the implant fabrication instruction set 161 to the implant fabrication device 160. If the stiffness measuring device 120 has provided a spine stiffness parameter to the control system 110, the planning module 150 incorporates the spine stiffness parameter into the deformation target data set by applying the overbend correction before transmitting the implant fabrication instruction set 161 to the implant fabrication device 160. The implant fabrication device 160 receives the implant fabrication instruction set 161 and physically deforms the orthopedic implant intraoperatively using sensor-feedback-controlled bending per each discrete segment. After attachment of the fabricated orthopedic implant to the person 101's spine, the intraoperative verification module 170 receives intraoperative spinal alignment data, compares the intraoperative spinal alignment data against the desired postoperative spinal alignment, and either verifies compliance or triggers the planning module 150 to generate an updated implant fabrication instruction set 161. If an updated implant fabrication instruction set 161 is generated, the implant fabrication device 160 re-processes the orthopedic implant accordingly, and the intraoperative verification module 170 repeats the verification after reattachment. This closed-loop architecture enables iterative intraoperative correction, such that the system 100 can detect segment-by-segment deviations between the targeted and actual postoperative alignments, quantify the resulting musculoskeletal force differentials, and determine whether the orthopedic implant should be removed, re-bent, and reattached before surgical closure.

Specifically, the measurement module 130 is the entry point of the computational pipeline and is configured to receive, as input, patient musculoskeletal data sets comprising spinal characteristic data objects derived at least from medical imaging of the person 101's spine. Patient musculoskeletal data sets, in this context, encompass any structured collection of quantitative anatomical and physiological data descriptive of a patient's musculoskeletal system, and spinal characteristic data objects are data structures encoding geometric, morphological, and biomechanical properties of the person 101's spinal column as extracted or derived from imaging or measurement devices. The medical imaging from which the spinal characteristic data objects are derived may include X-ray imaging (including biplanar imaging systems such as EOS), computed tomography (CT), and magnetic resonance imaging (MRI), among other modalities.

**In** some embodiments, the spinal characteristic data objects further comprise dynamic motion data sets from one or more inertial measurement units (IMUs) positioned on the person 101's vertebrae. The dynamic motion data sets being representative of patient-specific dynamic sagittal alignment captured during functional activities such as sit-to-stand transitions, gait, and flexion-extension movements. Where CT imaging is available, the spinal characteristic data objects may further encode volumetric bone density information from which a bone mineral density of the person 101 can be derived.

The spinal characteristic data may be supplemented by intraoperative measurements conducted by contact-based devices. The stiffness measuring device 120 is configured to apply a predetermined displacement or force onto the person 101's spine and measure a resulting force or displacement to derive, as output, a spine stiffness parameter. The spine stiffness parameter is a quantitative measure of the mechanical resistance of the person 101's spinal column to deformation at one or more vertebral levels, expressed for example as force per unit displacement (N/mm). The stiffness measurement can be performed intraoperatively after osteotomy and release procedures have been carried out, at which point the mechanical properties of the spine differ from their preoperative state due to vertebral structures having been cut, released, or otherwise modified.

In some embodiments, the stiffness measuring device 120 is implemented as a robotic arm that functions as a three-point loading apparatus, applying a controlled force at a specific vertebral level and measuring the resulting displacement, from which the local segmental stiffness is computed as force divided by displacement. The stiffness measuring device 120 may repeat this measurement at various vertebral levels along the instrumented spine to build a segmental stiffness profile. In some embodiments, the stiffness may additionally or alternatively be estimated from preoperative imaging data (e.g., CT and MRI), IMU-derived motion data, or a combination thereof. The spine stiffness parameter may be transmitted for use by the planning module 150 in applying the overbend correction.

In some embodiments, the measurement module 130 generates a three-dimensional reconstruction of the patient's spine from the detected landmark coordinates and segmentation outputs. Where biplanar radiographic imaging (e.g., EOS) is employed, the measurement module 130 reconstructs the three-dimensional spinal geometry by combining the two-dimensional landmark coordinates detected in both the anteroposterior and lateral radiographic views using stereo calibration parameters and epipolar geometry constraints to produce a three-dimensional spatial model comprising the position and orientation of each vertebral body in three-dimensional space. Where three-dimensional volumetric imaging (e.g., CT or MRI) is employed, the three-dimensional reconstruction is derived directly from the three-dimensional segmentation volume or the three-dimensional voxel coordinates of the detected anatomical landmarks. The three-dimensional reconstruction of the patient's spine is rendered via the user interface 180 as a rotatable, zoomable three-dimensional spine model that enables the operator to inspect the patient's spinal geometry from any viewing angle, including sagittal, coronal, axial, and oblique perspectives.

The three-dimensional reconstruction may be further used by the simulation module 140 as the baseline preoperative spinal geometry upon which the mathematical transformations for generating the candidate realignment configurations are applied, and by the planning module 150 in computing the desired three-dimensional rod profile from which the deformation target data set is derived.

The measurement module 130 processes the spinal characteristic data objects by executing image recognition and landmark detection algorithms. **In** some embodiments, trained artificial intelligence techniques may be employed to automatically identify vertebral body boundaries, endplate orientations, and pelvic landmarks within the medical imaging data.

In some embodiments, the trained artificial intelligence models employed by the measurement module 130 comprise one or more image processing algorithms and deep learning models trained on a corpus of annotated spinal imaging data. The annotated spinal imaging data comprises a plurality of medical images, such as biplanar radiographs, CT slices, or MRI slices, in which anatomical landmarks have been manually identified and labeled by clinicians. The anatomical landmarks include, without limitation, the four corners of each vertebral body (superior-anterior, superior-posterior, inferior-anterior, inferior-posterior), the center of each vertebral endplate, the sacral endplate, the center of each femoral head, and pelvic landmarks such as the anterior-superior iliac spine (ASIS) and the posterior-superior iliac spine (PSIS). Each annotated image in the corpus associates pixel coordinates or voxel coordinates of the anatomical landmarks with the corresponding medical image, forming a labeled training data set.

For vertebral body segmentation, the deep learning model may be configured as a semantic segmentation network that outputs a per-pixel classification map, wherein each pixel in the input medical image is assigned a class label corresponding to a specific vertebral body (e.g., T1, T2, ... L5), the sacrum, the pelvis, or background. The segmentation map enables the measurement module 130 to delineate the precise boundaries of each vertebral body, from which endplate orientations are computed as the angular orientation of the line connecting the anterior and posterior edges of each endplate.

The measurement module 130 further identifies, from the landmark coordinates and the computed segmental Cobb angles, a plurality of spinal curvature features comprising: a lumbar lordosis (LL) apex, defined as the vertebral body or intervertebral level at which the sagittal curvature of the lumbar spine reaches its maximum lordotic angulation; a thoracolumbar inflection point, defined as the vertebral level at which the spinal curvature transitions from thoracic kyphosis to lumbar lordosis in the sagittal plane, representing the kyphosis-to-lordosis transition; a thoracic kyphosis (TK) apex, defined as the vertebral body or intervertebral level at which the sagittal curvature of the thoracic spine reaches its maximum kyphotic angulation; a cervicothoracic inflection point, defined as the vertebral level at which the spinal curvature transitions from cervical lordosis to thoracic kyphosis in the sagittal plane; and a cervical segment apex, defined as the vertebral body or intervertebral level at which the sagittal curvature of the cervical spine reaches its maximum lordotic angulation.

The measurement module 130 identifies each apex and inflection point by analysing the sequence of segmental Cobb angles along the spinal column and detecting the vertebral levels at which the segmental Cobb angle reaches a local extremum (for apices) or at which the sign of the segmental Cobb angle reverses (for inflection points). The identified apices and inflection points are included within the plurality of spinopelvic alignment parameters output by the measurement module 130 and are rendered graphically via the user interface 180 as labelled markers superimposed on the graphical spine representations. The identified apices and inflection points may be further utilized by the simulation module 140 in generating the candidate realignment configurations and by the planning module 150 in computing the desired rod profile.

From the identified anatomical features and boundaries, the measurement module 130 derives, as output, a plurality of spinopelvic alignment parameters, which are quantitative descriptors of the spatial relationships between vertebral bodies, the sacrum, the pelvis, and the global sagittal and coronal balance of the person 101. The plurality of spinopelvic alignment parameters may number in excess of fifty discrete parameters and may comprise: pelvic incidence (PI), sacral slope (SS), pelvic tilt (PT), lumbar lordosis (LL: inflection point (IP)-S1 Cobb angle), L1-S1 including segmental proximal and distal measures such as L1-L4 and L4-S1, thoracic kyphosis (TK, including global TK: T1-IP Cobb angle and T4-T12), sagittal vertical axis (SVA), T1 pelvic angle (TPA or T1 PA), global tilt (GT), C7 ratio, L1 pelvic angle (L1PA), segmental pelvic angles at each vertebral level (e.g., L2PA, L3PA, L4PA, L5PA), PI minus LL (PI-LL), segmental Cobb angles at each intervertebral level from T1-T2 through L5-S1 in coronal, sagittal, and spatial views, and a Global Alignment and Proportion (GAP) score.

The measurement module 130 computes the plurality of spinopelvic alignment parameters by applying geometric analysis routines to the landmark coordinates. For example, at each intervertebral level, the segmental Cobb angle is computed as the angle between a first line defined by the superior endplate of the inferior vertebra and a second line defined by the inferior endplate of the superior vertebra; global alignment parameters such as lumbar lordosis (LL) are computed as the Cobb angle between the superior endplate of L1 and the superior endplate of S1 (i.e., L1-S1), or the angle between a perpendicular planes to the spine at inflection point and the superior endplate of S1; pelvic incidence (PI) is computed as the angle between a line perpendicular to the sacral endplate at its midpoint and a line connecting the midpoint of the sacral endplate to the center of the femoral heads; sacral slope (SS) is computed as the angle between the sacral endplate and a horizontal reference line; pelvic tilt (PT) is computed as the angle between a vertical reference line and a line connecting the midpoint of the sacral endplate to the center of the femoral heads; the sagittal vertical axis (SVA) is computed as the horizontal distance between a plumb line dropped from the centroid of the C7 vertebral body and the posterior-superior corner of the sacral endplate.

In some embodiments, the measurement module 130 may be operated semi-automatically, with user-assisted landmark identification via the user interface 180. The measurement module 130 stores the derived spinopelvic alignment parameters in a structured data object that can be exported for later retrieval, enabling the measurement to be performed once and reused across simulation and planning sessions.

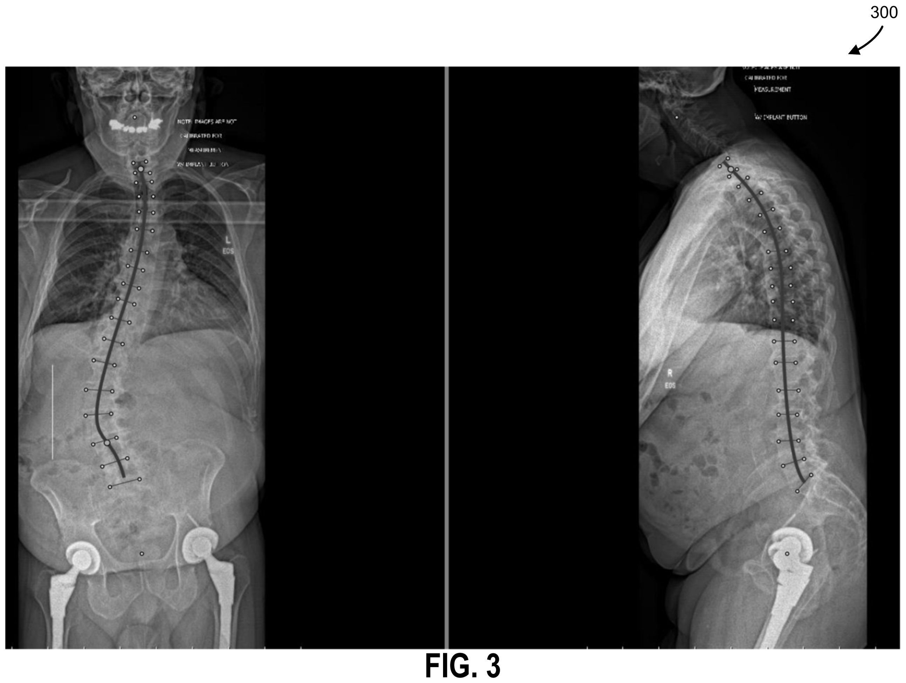

The measurement module 130 may render its outputs graphically via the user interface 180. **FIG.** 3 illustrates an example graphical representation 300 of a patient's spine as rendered by the measurement module 130 via the user interface 180. The graphical representation 300 depicts the vertebral levels from the upper thoracic spine (e.g., T1) through the lumbar spine (e.g., L5) as individually identifiable vertebral body elements.

The graphical representation 300 is generated from the patient musculoskeletal data sets, specifically from the spinal characteristic data objects derived from medical imaging of the person 101's spine. In addition to a main curve tracing the centerline of the person 101's spine in each planar view, alphanumeric labels (transverse to the main curve) are overlaid on the graphical representation 300 at the position of each detected vertebral body, each label identifying the corresponding vertebral level (e.g., T1, T2, T3, ... L3, L4, L5). The red labels are generated by the measurement module 130 as a result of the image recognition and landmark detection algorithms applied to the patient's medical imaging data, such that the position of each red label corresponds to the pixel or spatial coordinates of the detected vertebral body in the underlying medical image.

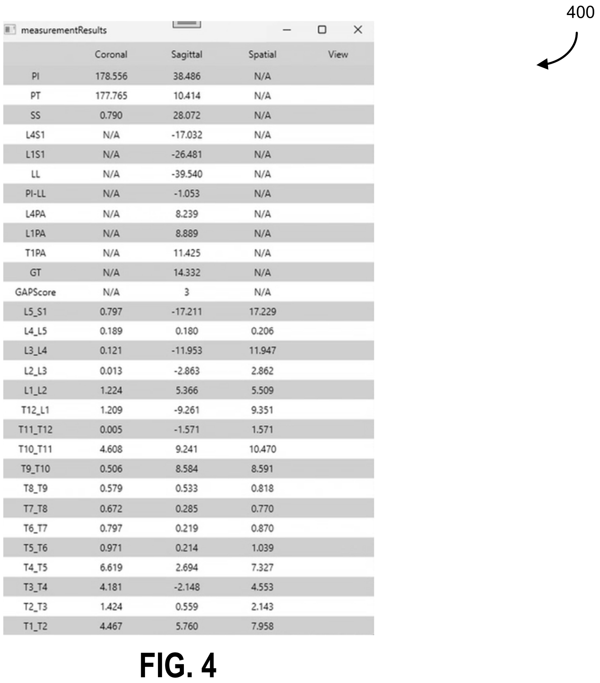

**FIG. 4** illustrates an example parameter display 400 showing the plurality of spinopelvic alignment parameters derived by the measurement module 130 from the patient musculoskeletal data sets. The example parameter display 400, as rendered on the user interface 180, presents the spinopelvic alignment parameters in a tabular format organized by coronal, sagittal, and spatial views. In addition to the data fields as shown in the parameter display 400, the plurality of spinopelvic alignment parameters may number in excess of fifty discrete parameters, comprising: pelvic incidence (PI), sacral slope (SS), pelvic tilt (PT), lumbar lordosis (LL, including segmental measures such as L4-S1 and L1-S1), thoracic kyphosis (TK, including global TK and T4-T12), sagittal vertical axis (SVA), T1 pelvic angle (TPA or T1PA), global tilt (GT), C7 ratio, L1 pelvic angle (L1PA), segmental pelvic angles at each vertebral level (e.g., L2PA, L3PA, L4PA, L5PA), PI minus LL (PI-LL), and GAP scores.

The parameter display 400 further shows segmental Cobb angles at each intervertebral level from T1-T2 through L5-S1 in coronal, sagittal, and spatial views, providing a comprehensive quantitative characterization of the patient's baseline spinal geometry as derived by the measurement module 130. Each spinopelvic alignment parameter is a computed numerical value derived from geometric analysis of the spinal characteristic data objects. The derivation may be performed automatically by the computer processor of the control system 110 executing image recognition and landmark detection algorithms, or in some embodiments, may be performed semi-automatically with user-assisted landmark identification via the user interface 180. In some embodiments, the measurement module 130 derives the spinopelvic alignment parameters automatically using trained artificial intelligence techniques applied to the medical imaging data. The measurement data shown in the parameter display 400 can be exported and stored for later retrieval, enabling the measurement to be performed once and reused across simulation and planning sessions.

Referring back to **FIGs 1-2**, the simulation module 140 is the second stage of the computational pipeline and receives, as input, the plurality of spinopelvic alignment parameters output by the measurement module 130. The simulation module 140 is configured to simulate, based on the plurality of spinopelvic alignment parameters, one or more candidate realignment configurations, each candidate realignment configuration representing a computationally generated data structure encoding a set of segmental changes required across a plurality of vertebral levels to achieve a desired postoperative spinal alignment.

The simulation module 140 generates the candidate realignment configurations by applying transformations to the baseline preoperative spinal geometry, such as the derived spinopelvic alignment parameters from the measurement module 130. For a given upper instrumented vertebra (UIV) and lower instrumented vertebra (LIV) selection, each alignment classification criterion produces a distinct set of target spinopelvic alignment parameters, resulting in a distinct candidate realignment configuration. The plurality of alignment classification criteria comprises one or more of: a Global Alignment and Proportion (GAP) classification, an age-adjusted alignment, a Roussouly classification, a segmental alignment, a T4-L1-axis alignment, and an SRS-Lenke-Aubin three-dimensional classification of adolescent idiopathic scoliosis. The operator may specify the UIV and LIV selection, and may optionally override individual target spinopelvic alignment parameter values (e.g., sacral slope) within a given classification criterion via the user interface 180.

For each candidate realignment configuration, the simulation module 140 further computes estimated musculoskeletal forces at one or more vertebral levels. The estimated musculoskeletal forces comprise at least shear forces (forces acting tangentially to the vertebral endplate surfaces) and compression forces (forces acting normal to the vertebral endplate surfaces) at each intervertebral level, and in some embodiments further comprise bending moments, lateral shear forces, and disc-level forces at the intervertebral disc junction between adjacent vertebrae. The estimated musculoskeletal forces are determined by solving a patient-specific musculoskeletal model such as an inverse dynamics model. An inverse dynamics model may account for the person 101's sex, weight, height, and spinal curvature as input parameters to the computation. Where the spinal characteristic data objects include dynamic motion data sets from one or more IMUs, the simulation module 140 integrates the dynamic motion data sets into the musculoskeletal force computation such that the estimated musculoskeletal forces reflect not only the static standing alignment but also the dynamic forces that the spine and the attached orthopedic implant will experience during the person 101's habitual movement patterns.

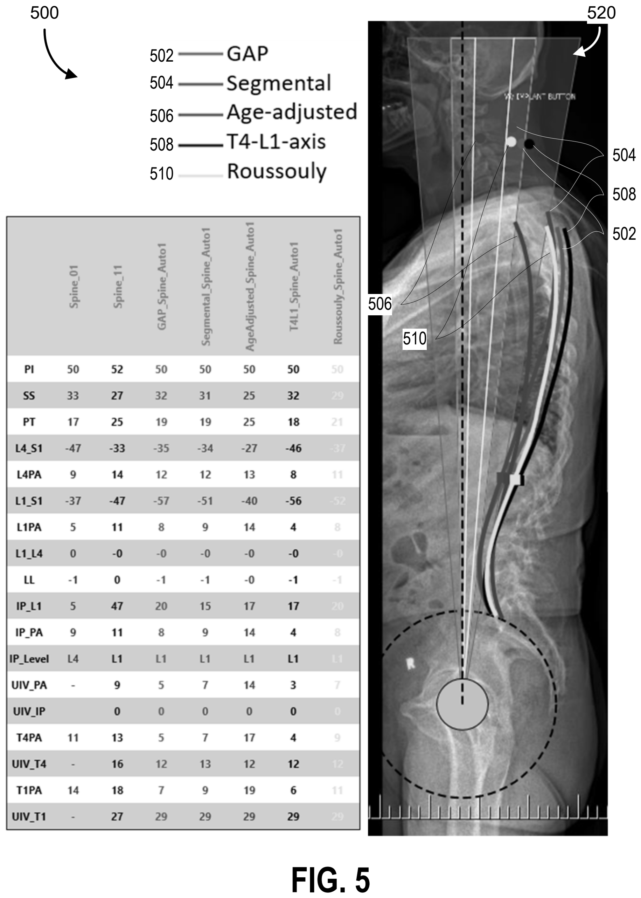

The simulation module 140 further computes a risk factor of mechanical complication for each candidate realignment configuration, the risk factor computed based on the estimated musculoskeletal forces at one or more vertebral levels in combination with a bone mineral density of the person 101. The risk factor of mechanical complication comprises an estimated probability in the occurrence of one or more of proximal junctional failure (PJF), proximal junctional kyphosis (PJK), rod fracture, sacral fracture, adjacent segment disease, and pseudoarthrosis. The simulation module 140 is further configured to render, via the user interface 180, a cone of economy visualisation indicating musculoskeletal expenditure for each candidate realignment configuration. The outputs of the simulation module 140 thus comprise: the one or more candidate realignment configurations with their corresponding simulated postoperative spinal geometries; the estimated musculoskeletal forces at each vertebral level for each candidate realignment configuration; the risk factor of mechanical complication for each candidate realignment configuration; and the cone of economy visualisation. The simulation module 140 renders these outputs graphically via the user interface 180, as described below with reference to **FIGs. 5-10****.**

In some embodiments, the simulation module 140 further utilises a trained artificial intelligence model to predict postoperative alignment parameters of the unfused spinal segments, the artificial intelligence model receiving as input the preoperative spinal geometry, the selected candidate realignment configuration including a fused segment, and patient demographic data, and outputting predicted values for alignment parameters in the unfused spine such as postoperative sacral slope and unfused segment compensation such as postoperative T1 pelvic angle and postoperative T1-UIV Cobb angle.

The trained artificial intelligence model employed by the simulation module 140 for unfused spine prediction may be a supervised learning model trained on a retrospective clinical data set comprising preoperative and postoperative spinal alignment measurements from a plurality of patients who have previously undergone spinal fusion surgery. The retrospective clinical data set comprises, for each patient in the data set, a set of preoperative input features and a set of postoperative output labels. The preoperative input features may include: preoperative spinopelvic alignment parameters such as preoperative sacral slope, preoperative pelvic tilt, pelvic incidence, preoperative lumbar lordosis (e.g., preoperative L1-S1 angle, preoperative L4-S1 angle), preoperative thoracic kyphosis, preoperative sagittal vertical axis, and preoperative T1 pelvic angle; surgical planning parameters such as the selected candidate realignment configuration including the postoperative L1-S1 angle, the postoperative L1PA, the UIV and LIV selections, and the types and locations of corrective procedures performed (e.g., posterior column osteotomy, pedicle subtraction osteotomy, interbody cage insertion); and patient demographic data including the patient's age, sex, height, weight, and body mass index. The postoperative output labels comprise the measured postoperative values of alignment parameters in the unfused spinal segments, including postoperative sacral slope, postoperative T1 pelvic angle, postoperative pelvic tilt, and postoperative thoracic kyphosis, as measured from postoperative imaging (e.g., postoperative standing biplanar radiographs) obtained after the patient's spine has reached a stable postoperative alignment.

At inference time, the simulation module 140 constructs an input feature vector for a given person 101 by populating the preoperative input features from the plurality of spinopelvic alignment parameters derived by the measurement module 130, populating the surgical planning parameters from the selected candidate realignment configuration, and populating the patient demographic data from the patient musculoskeletal data sets. The input feature vector is provided to the trained artificial intelligence model, which outputs predicted postoperative values for the unfused spine alignment parameters, including predicted postoperative sacral slope, predicted postoperative T1 pelvic angle, predicted postoperative T1-UIV Cobb angle, and predicted postoperative pelvic tilt.

Since the sacral slope and pelvic tilt are pelvic parameters that are not directly controllable by the surgical intervention, the predicted unfused spine alignment parameters are used by the control system 110 to refine the implant fabrication instruction set 161. Specifically, the planning module 150 incorporates the predicted postoperative unfused spine alignment into the rod profile computation by adjusting the target spinal geometry to account for the anticipated compensatory response. For example, if the trained artificial intelligence model predicts that the patient's sacral slope will decrease by a certain number of degrees relative to the value assumed by the selected alignment classification criterion, the planning module 150 adjusts the deformation target data set such that the orthopedic implant, when fabricated and attached, will produce the desired postoperative spinal alignment inclusive of the predicted compensatory pelvic response.

**FIG. 5** illustrates a simulation display 500 generated by the simulation module 140, showing a plurality of candidate realignment configurations generated according to a plurality of alignment classification criteria. Alignment classification criteria are clinically established frameworks that define target postoperative spinopelvic geometries. In some embodiments, the plurality of alignment classification criteria comprises one or more of: the Global Alignment and Proportion (GAP) classification (rendered as the GAP alignment 502), which evaluates proportional relationships between PI, SS, and GT and assigns a numerical GAP score indicative of alignment quality, where a lower GAP score indicates a more proportional alignment; the Age-adjusted alignment (rendered as the Age-adjusted alignment 506), which modifies target spinopelvic values based on the patient's age to account for normative age-related changes in spinal curvature, such as increased thoracic kyphosis and reduced lumbar lordosis in elderly patients; the Roussouly classification (rendered as the Roussouly alignment 510), which categorises spines into morphological types (Types 1 through 4) based on the relationship between sacral slope and the distribution of lumbar lordosis, each Roussouly type defining a distinct target lumbar curve shape (e.g., Roussouly Type III with SS = 35); the Segmental alignment (rendered as the Segmental alignment 504), which targets normative Cobb angle values at each individual intervertebral level independently; and the T4-L1-axis alignment (rendered as the T4-L1-axis alignment 508), which uses the spatial relationship between the T4 and L1 vertebral bodies as the governing alignment parameter for the correction.

Each candidate realignment configuration specifies, for each vertebral level between a selected upper instrumented vertebra (UIV) and lower instrumented vertebra (LIV), a target intervertebral angle that, in aggregate, yields the desired postoperative spinal alignment. The desired postoperative spinal alignment is the target spinal geometry that the surgeon selects from among the candidate realignment configurations as the objective for the spinal fusion surgery, and the orthopedic implant is fabricated to achieve the desired postoperative spinal alignment when attached.

The simulation display 500 further renders, for each candidate realignment configuration, a shaded triangular cone of economy region 520 superimposed on the corresponding graphical spine representation. Each cone of economy region 520 is a triangular area rendered in the sagittal plane that represents a spatial envelope within which the patient's centre of gravity can be maintained with minimal muscular effort. Each cone of economy region 520 is bounded by two diverging lines extending upward from the patient's base of support, forming a triangular zone in which the projected centre of gravity of the person 101 is situated. The position of the projected centre of gravity relative to the centre of the cone of economy region 520 is an indicator of musculoskeletal expenditure: a projected centre of gravity that falls near the centre of the cone of economy region 520 indicates that the patient can maintain an upright standing posture with minimal compensatory muscular effort, whereas a projected centre of gravity that falls near a boundary or outside the cone of economy region 520 indicates that greater muscular effort is required to maintain postural balance, resulting in higher musculoskeletal expenditure, accelerated muscular fatigue, and a greater risk of postural decompensation.

The cone of economy region 520 for each of the realignment configuration enables the operator to compare how each candidate configuration positions the patient's centre of gravity within or relative to the safe postural zone, and to make informed decisions regarding the optimal plan of treatment. For example, if the GAP alignment 502 positions the projected centre of gravity most centrally within its cone of economy region 520, it would indicate that the GAP alignment may represent the most biomechanically favorable candidate realignment configuration for the patient. The cone of economy region 520 thus provides a comparative tool by which the operator can assess and select the optimal candidate realignment configuration for a given patient. Embodiments of the simulation module 140 may be configured to automatically recognize an optimal realignment configuration based on a candidate configuration's compliance to the zone of economy.

The simulation display 500 further includes a tabular comparison of spinopelvic alignment parameters for each candidate realignment configuration, enabling quantitative side-by-side assessment. The tabular comparison shows, for each of the GAP alignment 502, the Segmental alignment 504, the Age-adjusted alignment 506, the T4-L1-axis alignment 508, and the Roussouly alignment 510, the values for PI, SS, PT, L4-S1, L4PA, L1-S1, L1PA, LL, UIV-PA, T4PA, T1PA, and other parameters. The simulation module 140 generates the candidate realignment configurations by applying mathematical transformations to the baseline preoperative spinal geometry, where the baseline preoperative spinal geometry is the spatial representation of the patient's spine as measured from the spinal characteristic data objects. For a given UIV and LIV selection (e.g., UIV = T10, LIV = S1), each alignment classification criterion produces a distinct set of target spinopelvic alignment parameters, resulting in a distinct candidate realignment configuration.

In one embodiments, the alignment classification criterion is the SRS-Lenke-Aubin three-dimensional classification of adolescent idiopathic scoliosis, which categorises adolescent idiopathic scoliosis deformities based on three components: a primary curve type (Types 1 through 6) determined by the location and structural characteristics of the major and minor coronal curves, a lumbar spine modifier (A, B, or C) determined by the relationship of the centre sacral vertical line (CSVL) to the lumbar apical vertebra, and a sagittal thoracic modifier (-, N, or +) determined by the magnitude of T5-T12 thoracic kyphosis relative to normative thresholds. The measurement module 130 provides the coronal Cobb angle measurements, the identification of the apical vertebrae, and the sagittal thoracic kyphosis measurement from which the simulation module 140 derives the SRS-Lenke-Aubin classification for a given patient, and the simulation module 140 generates the corresponding candidate realignment configuration by applying the classification-specific correction targets for the primary curve and any structural minor curves.

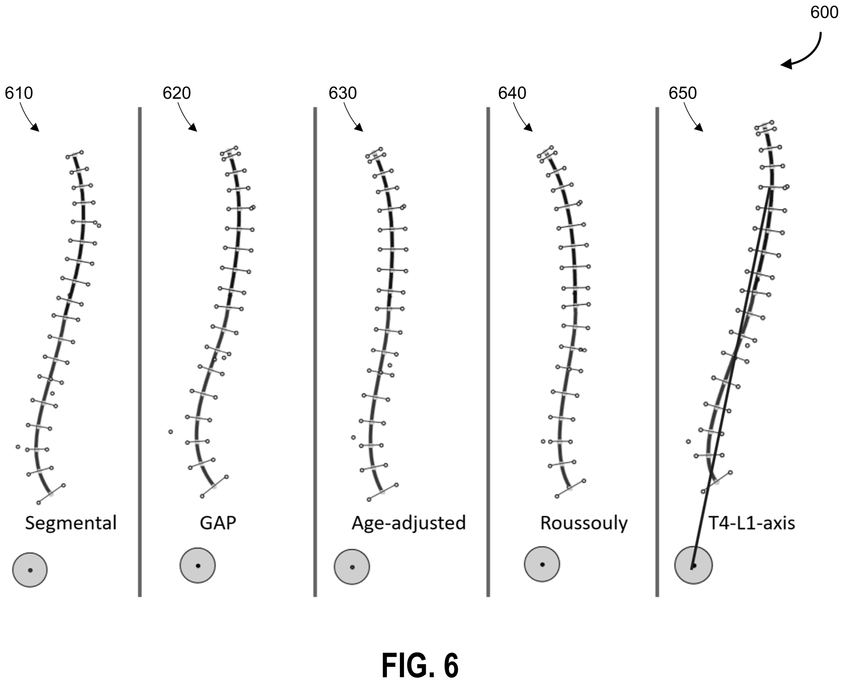

**FIG.** 6 illustrates simulation results 600 rendered by the simulation module 140, showing the simulated postoperative spinal geometries produced by each of the alignment classification criteria alongside the preoperative and actual postoperative spinal geometries. The simulation results display 600 renders a plurality of individual spine visualisations: a Preoperative spine visualisation 602, an Age-adjusted spine visualisation 604, a GAP spine visualisation 606, a Roussouly spine visualisation 608, a Segmental spine visualisation 610, a T4-L1-axis spine visualisation 612, and a Postoperative spine visualisation 614. Each of the visualization depicts the simulated spinal geometry that would result from applying the corresponding alignment classification criterion to the patient's baseline preoperative spinal geometry for a given UIV and LIV selection. The simulation results 600 and simulation display 500 enable the operator to visually assess how each alignment classification criterion reshapes the patient's spinal curvature, thereby facilitating quantitative selection of the candidate realignment configuration that best serves the clinical objectives for the patient.

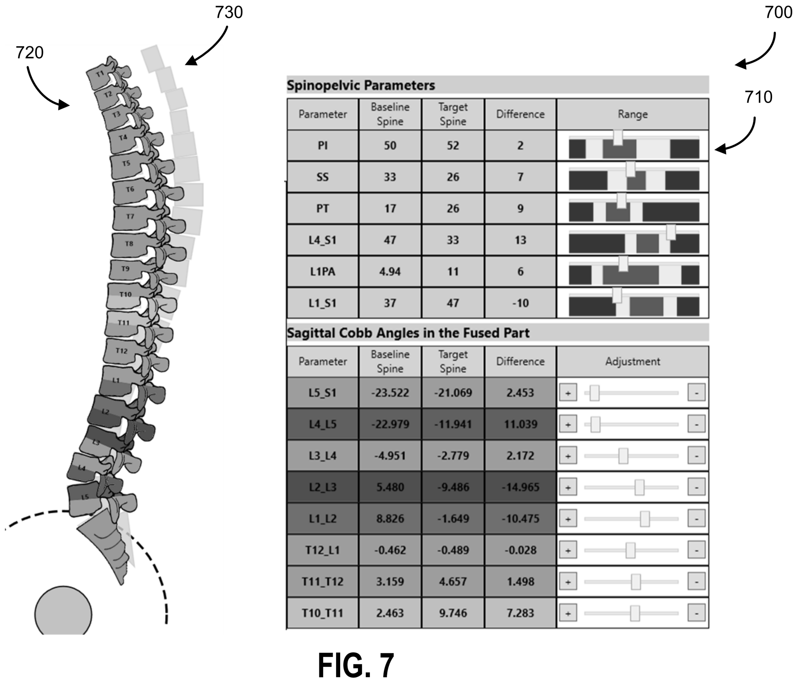

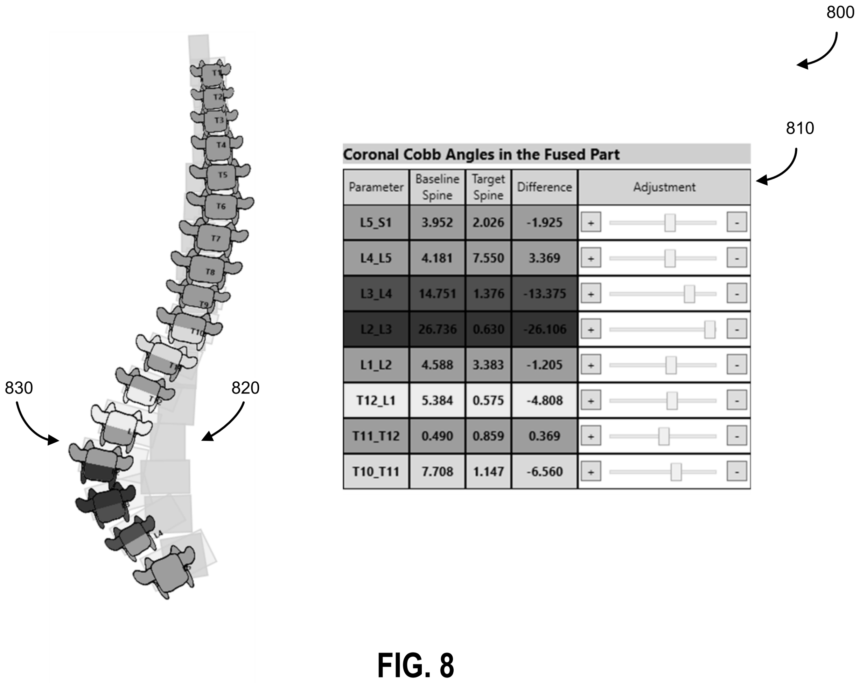

**FIG. 7** and **FIG. 8** illustrate interactive displays that bridge the simulation module 140 and the planning module 150, serving a dual function within the computational pipeline of the system 100. The simulation module 140 generates the initial graphical content of the displays, including the color-coded spine visualisations, the spinopelvic parameter tables, and the target Cobb angle values derived from the selected candidate realignment configuration, and renders this content in two planes as simulations 700 and 800. The planning module 150 then operates upon these displays by receiving operator-specified corrective procedures at individual vertebral levels and updating the color-coded spine visualisations and parameter tables in real time to reflect the cumulative effect of the planned corrections. Together, **FIG. 7** and **FIG. 8** thus represent the interactive working environment in which the operator transitions from having selected a candidate realignment configuration (the output of the simulation module 140) to having defined a fully specified corrective surgical plan (the input consumed by the planning module 150 to generate the implant fabrication instruction set 161).

**FIG. 7** illustrates a sagittal simulation 700 rendered for display at the user interface 180. The simulation module 140 generates the sagittal simulation 700 by computing, for each vertebral level in the fused region between the selected UIV and LIV, a baseline sagittal Cobb angle derived from the patient's preoperative spinal geometry (baseline spine) and a target sagittal Cobb angle derived from the selected candidate realignment configuration (target spine). The sagittal simulation 700 includes a spinopelvic parameters table 710 showing the baseline spine values and the target spine values for key spinopelvic alignment parameters including PI, SS, PT, L4-S1, L1PA, and L1-S1, together with the numerical difference between the baseline and target values for each parameter. The spinopelvic parameters table 710 is populated by the simulation module 140 from the plurality of spinopelvic alignment parameters derived by the measurement module 130 and the corresponding target values computed by the simulation module 140 for the selected alignment classification criterion.

The sagittal simulation 700 further includes graphical spine representations comprising a baseline spine visualisation 720 and a target spine visualisation 730, rendered side by side in the sagittal plane. The simulation module 140 generates the baseline spine visualisation 720 from the patient's preoperative spinal geometry (the spatial representation of the person 101's spine as measured from the spinal characteristic data objects) and generates the target spine visualisation 730 (the simulated postoperative spinal geometry of the selected candidate realignment configuration). At each vertebral level in the fused region, a colored bar is superimposed on the baseline spine visualisation 720, the color of the colored bar encoding the magnitude of difference between the baseline sagittal Cobb angle and the target sagittal Cobb angle at that vertebral level. The computer processor is configured to render, for display in real time as the baseline spine visualisation 720 is modified toward the target spine visualisation 730, segmental Cobb angles at each vertebral level in the sagittal plane, wherein each vertebra is rendered with a color corresponding to a magnitude of difference between a baseline Cobb angle and a target Cobb angle. The color encoding may employ a graduated color scale, for example ranging from green (indicating that the baseline Cobb angle at that vertebral level is within a predetermined tolerance of the target Cobb angle) through yellow (indicating a moderate deviation) to red (indicating a large deviation requiring substantial corrective intervention at that vertebral level). This color-coded rendering enables the operator to identify, at a glance, which vertebral levels in the fused region require the greatest corrective intervention and which are already close to the target alignment, without requiring manual numerical comparison of individual Cobb angle values.

The sagittal simulation 700 further provides per-level adjustment controls (e.g., "+" and "-" buttons) adjacent to each vertebral level in the fused region, enabling the operator to apply corrective procedures at individual vertebral levels and observe the resulting change in color-coded deviation in real time. The per-level adjustment controls are interactive graphical elements rendered by the user interface 180 that, when actuated by the operator, transmit a corrective procedure instruction to the planning module 150. The planning module 150 receives the corrective procedure instruction and recalculates the baseline sagittal Cobb angle at the specified vertebral level to account for the angular change introduced by the corrective procedure. The planning module 150 then updates the color of the colored bar at that vertebral level in the baseline spine visualisation 720 in real time to reflect the reduced (or eliminated) magnitude of difference between the recalculated baseline Cobb angle and the target Cobb angle, providing immediate visual feedback as to the cumulative effect of the planned corrections.

The sagittal simulation 700 enables the application of corrective procedures at individual vertebral levels. For example, the operator may specify a posterior column osteotomy (PCO), a pedicle subtraction osteotomy (PSO), or an interbody cage insertion at a particular level using the per-level adjustment controls in the sagittal simulation display 700. A posterior column osteotomy involves resection of the posterior bony elements (e.g., facet joints, lamina, spinous process) at a given vertebral level, which increases the segmental Cobb angle at that level by a characteristic angular increment, typically in the range of approximately 5 to 10 degrees per level. A pedicle subtraction osteotomy involves a wedge-shaped resection through the vertebral body itself, producing a larger angular correction, typically in the range of approximately 25 to 35 degrees at the osteotomy level. An interbody cage insertion involves placement of a structural spacer in the intervertebral disc space, which restores or increases the segmental Cobb angle at that level by an amount determined by the cage height and geometry. As each corrective procedure is applied via the per-level adjustment controls, the planning module 150 recalculates the predicted angular effect of the procedure on the segmental Cobb angle at the specified vertebral level and updates the color of the colored bar at that vertebral level in the baseline spine visualisation 720 in real time to reflect the reduced magnitude of difference between the baseline Cobb angle and the target Cobb angle.

The real-time color-coded rendering enables the user interface 180 to communicate, at each vertebral level, the type and magnitude of corrective procedure required without requiring manual calculation. The spinopelvic parameters table 710 also updates in real time to reflect the effect of each applied procedure on the global spinopelvic alignment parameters, because modifying the segmental Cobb angle at one or more vertebral levels propagates into changes in the global parameters such as lumbar lordosis, sagittal vertical axis, and T1 pelvic angle. The real-time co-updating of both the color-coded spine visualisation and the spinopelvic parameters table 710 ensures that the operator can simultaneously assess the local segmental effect and the global alignment effect of each corrective procedure as it is planned.

**FIG. 8** illustrates a coronal simulation 800 that operates in an analogous manner to the sagittal simulation 700 of **FIG. 7**, but in the coronal plane. The simulation module 140 generates the coronal simulation display 800 by computing, for each vertebral level in the fused region, a baseline coronal Cobb angle derived from the patient's preoperative spinal geometry and a target coronal Cobb angle derived from the selected candidate realignment configuration. The coronal simulation 800 includes a coronal Cobb angles table 810 showing, for each vertebral level in the fused region (e.g., L5-S1 through T10-T11), the baseline coronal Cobb angle, the target coronal Cobb angle, and the numerical difference between them. The coronal simulation 800 further includes graphical spine representations for a baseline spine 830 and a target spine 820. Colored bars may employ the same graduated color scale as the sagittal simulation 700 to indicate the coronal deviation at each vertebral level, and per-level adjustment controls.

As with the sagittal simulation 700, the per-level adjustment controls in the coronal simulation 800 transmit corrective procedure instructions to the planning module 150, which recalculates the baseline coronal Cobb angle at the specified vertebral level to account for the angular change introduced by the corrective procedure and updates the baseline spine 830 and the coronal Cobb angles table 810 in real time. The planning module 150 integrates the coronal corrections specified via the coronal simulation display 800 with the sagittal corrections specified via the sagittal simulation 700 such that the final corrected spinal geometry from which the implant fabrication instruction set 161 is generated accounts for both sagittal and coronal deformity correction simultaneously. This dual-plane planning capability is important for patients with combined sagittal and coronal deformity, such as degenerative lumbar scoliosis, where the orthopedic implant must be contoured to correct curvature in both planes simultaneously. The planning module 150 combines the sagittal and coronal corrective procedure selections into a unified three-dimensional corrected spinal geometry, from which the desired rod profile is derived and decomposed into the deformation target data set as described further below.

The interaction between the simulation module 140 and the planning module 150 in the context of the simulations 700 and 800 may be understood as follows. The simulation module 140 is responsible for generating the initial state of the simulation. The simulation module 140 computes the initial values by applying the numerical transformation associated with the selected alignment classification criterion to the patient's preoperative spinal geometry, as described above with reference to **FIGs. 5-6****.** The planning module 150 receives operator-specified corrective procedures via the per-level adjustment controls, recalculates the segmental Cobb angles and global spinopelvic alignment parameters to account for the specified corrective procedures, and updates the color-coded bars and the spinopelvic parameters table in real time. Once the operator has completed specifying corrective procedures, the planning module 150 captures the corrected spinal geometry as the final desired postoperative spinal alignment and proceeds to generate the implant fabrication instruction set 161 comprising the deformation target data set.

A further interaction between the simulation module 140 and the planning module 150 may be to predict a risk of mechanical failure after implantation and to mitigate the risk accordingly in the fabrication process. Such risk analysis is a necessary component of the preoperative planning pipeline because the orthopedic implant, once attached to pedicle screws along the instrumented vertebral levels, fundamentally alters the distribution of mechanical forces across the patient's spinal column. The rod implant constrains the instrumented vertebral segments into a fixed postoperative geometry, and the vertebral levels immediately above and below the instrumented region must absorb the transition in mechanical loading between the rigidly instrumented spine and the mobile uninstrumented spine. This concentration of forces, especially at the UIV and UIV+1 levels, creates a biomechanical vulnerability that is the primary driver of postoperative mechanical complications, including proximal junctional failure, proximal junctional kyphosis, rod fracture, sacral fracture, adjacent segment disease, and pseudoarthrosis. Without quantitative force analysis prior to implant fabrication, the surgeon has no objective basis for assessing whether a given candidate realignment configuration will produce forces at the critical vertebral levels that exceed the structural capacity of the patient's bone and soft tissue, particularly in patients with reduced bone mineral density due to osteoporosis or osteopenia.

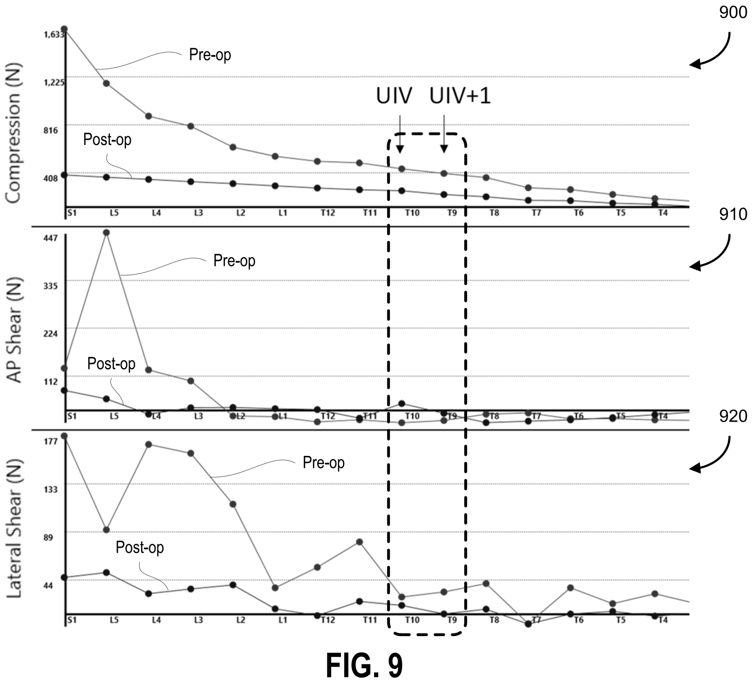

**FIG. 9** illustrates force estimates generated by the simulation module 140 via the user interface 180, showing the estimated musculoskeletal forces for the preoperative and postoperative spinal alignments. The force estimates include a compressive force chart 900 showing compression forces in Newtons at each vertebral level from L5 to T4, with the UIV and UIV+1 levels explicitly indicated. The compressive force chart 900 renders preoperative compression force values and postoperative compression force values as overlaid data series, enabling the operator to assess how the candidate realignment configuration changes the compressive loading at each vertebral level relative to the patient's baseline preoperative state. **FIG. 9** further includes an AP shear chart 910 showing anteroposterior shear forces at each vertebral level, and a lateral shear chart 920 showing lateral shear forces at each vertebral level, each with preoperative and postoperative values overlaid. The AP shear chart 910 and the lateral shear chart 920 enable quantitative comparison of the shear loading at the UIV and UIV+1 levels across the different candidate realignment configurations.

The three modes of loading rendered in **FIG. 9** each corresponds to a distinct biomechanical loading mechanism that drives specific modes of postoperative mechanical failure. Compression forces act normal to the vertebral endplate surfaces and are the primary determinant of vertebral body fracture risk: when the postoperative compression force at the UIV or UIV+1 level exceeds the compressive strength of the vertebral body (which is a function of the patient's bone mineral density), the endplate or cancellous core of the vertebral body may fail, resulting in proximal junctional failure. Anteroposterior shear forces act tangentially to the vertebral endplate surfaces in the sagittal plane and are the primary determinant of proximal junctional kyphosis risk: elevated AP shear forces at the UIV level cause progressive angular displacement between the UIV and UIV+1 vertebrae, manifesting as a pathological increase in kyphotic angulation at the proximal junction of the instrumented construct. Lateral shear forces act tangentially to the vertebral endplate surfaces in the coronal plane and contribute to asymmetric loading of the vertebral body and pedicle screws, which is a contributing factor to screw loosening, screw pull-out, and rod fracture at vertebral levels where the lateral shear force exceeds the fixation strength of the pedicle screw-bone interface. By rendering all three force categories across the full span of instrumented and adjacent vertebral levels, the simulation module 140 provides the operator with a comprehensive biomechanical profile from which the risk of each mode of mechanical failure can be assessed.

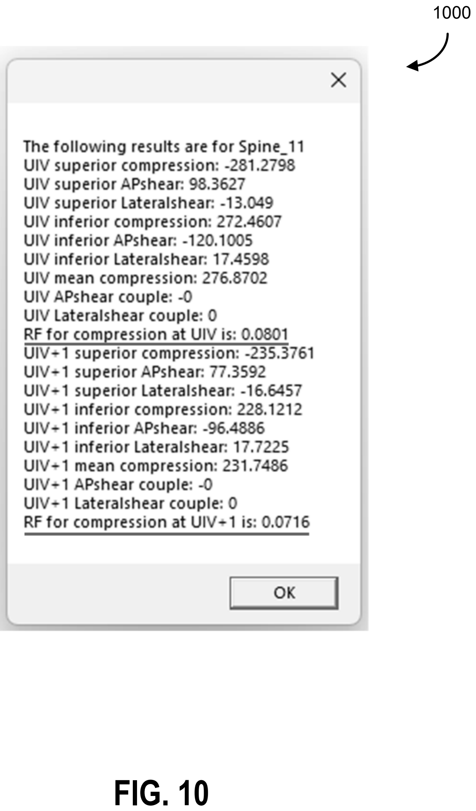

**FIG.** 10 illustrates a risk factor computation output 1000 generated by the simulation module 140 for display at the user interface 180, showing the detailed estimated musculoskeletal forces at the UIV and UIV+1 levels for a selected candidate realignment configuration. The risk factor computation output 1000 provides a focused, granular breakdown of the force data summarised in the force comparison of **FIG. 9**, isolating the UIV and UIV+1 levels for their heightened concentration of force and, consequently, the highest probability of postoperative mechanical failure. The risk factor computation output 1000 comprises, for each of the UIV and UIV+1 levels: superior compression (the compressive force acting on the superior endplate of the vertebral body), superior AP shear (the anteroposterior shear force acting on the superior endplate), superior lateral shear (the lateral shear force acting on the superior endplate), inferior compression (the compressive force acting on the inferior endplate), inferior AP shear (the anteroposterior shear force acting on the inferior endplate), inferior lateral shear (the lateral shear force acting on the inferior endplate), mean compression (the average of the superior and inferior compressive forces, representing the overall compressive loading on the vertebral body), AP shear couple (the difference between the superior and inferior AP shear forces, representing the rotational moment in the sagittal plane that tends to induce angular displacement between adjacent vertebrae), and lateral shear couple (the difference between the superior and inferior lateral shear forces, representing the rotational moment in the coronal plane).

The risk factor computation output 1000 further includes a computed risk factor (RF) for compression at the UIV (e.g., RF = 0.0801) and a computed risk factor for compression at the UIV+1 (e.g., RF = 0.0716). The risk factor of mechanical complication is a computationally derived numerical value representing an estimated probability of the occurrence of one or more postoperative mechanical failures. The risk factor of mechanical complication is computed based on the estimated musculoskeletal forces at one or more vertebral levels in combination with a bone mineral density of the patient. Each mode of mechanical failure comprised within the risk factor of mechanical complication is linked to a specific force category or combination of force categories as specified above.

In some embodiments, the simulation module 140 computes the risk factor by comparing the estimated musculoskeletal forces, particularly shear forces at the UIV and UIV+1 levels, against threshold values derived from a retrospective cohort analysis. In some embodiments, the computer processor further accounts for instrumental failure modes, including rod fracture risk computed based on the rod material properties (e.g., CoCr versus titanium alloy), the rod dimensions (e.g., diameter of 5.5 mm or 6.0 mm), and the computed cyclic loading expected from the patient's daily motion activities. The simulation module 140 may further compute screw pull-out risk and screw loosening risk based on a combination of the computed vertebral loading at each instrumented level and the bone mineral density at the corresponding vertebral bodies, including the screw insertion angle relative to the vertebral boundary surfaces. In some embodiments, the processor generates a fracture risk factor for each vertebral level based on a finite element analysis of the vertebral body, using segmented three-dimensional vertebral geometry derived from CT imaging with known material properties of the implant.

Referring back to **FIGs 1-2**, the planning module 150 is the third stage of the computational pipeline and receives, as input, a selected candidate realignment configuration from the simulation module 140 together with the corresponding simulated postoperative spinal geometry. The planning module 150 is further configured to receive, via the user interface 180, operator-specified corrective procedures at individual vertebral levels within the instrumented region. The corrective procedures may include posterior column osteotomy (PCO), pedicle subtraction osteotomy (PSO), interbody cage insertion, and local vertebral rotation, each of which modifies the segmental Cobb angle at the specified vertebral level. The planning module 150 renders, in real time via the user interface 180, the effect of each applied corrective procedure on the baseline spinal geometry, including color-coded bars indicating the degree of deviation between a baseline Cobb angle and a target Cobb angle at each vertebral level in sagittal and coronal planes.

Upon completion of the operator's corrective procedure selections, the planning module 150 generates, as output, an implant fabrication instruction set 161 comprising an implant deformation target data set. The deformation target data set defines, for each discrete segment of the orthopedic implant, three parameters: a target deformation angle, specifying the magnitude of plastic deformation in degrees to be applied at that discrete segment; a target deformation orientation, specifying the rotational plane about the longitudinal axis of the orthopedic implant in which the bend is to be applied; and a corresponding segment length, specifying the linear distance in millimetres along the longitudinal axis of the orthopedic implant between successive bend points.

The planning module 150 derives the deformation target data set by performing a numerical decomposition of the desired rod profile, turning a continuous three-dimensional curve representing the desired shape the orthopedic implant into a finite sequence of discrete bending operations. The decomposition may employ freeform curve representations including but not limited to Bézier curves, B-spline curves, or non-uniform rational B-spline (NURBS) representations. Using the decomposed implant shape, the planning module 150 generates a rod profile output comprising rod length, curve length up to the inflection point, per-level Cobb angles, interbody angles, and PCO angles.

In some embodiments, the planning module 150 generates the implant fabrication instruction sets 161 constrained by the specific modes of operation and physical characteristics of the implant fabrication device 160 that will execute the instruction set. The planning module 150 may receive, store, or have preconfigured therein a device capability profile comprising one or more of: a bending configuration type of the bending assembly (e.g., symmetrical three-point bending with two moving rollers and one fixed roller, or asymmetrical three-point bending with two fixed rollers and one moving roller); a roller geometry specification defining the diameter, spacing, and contact profile of the rollers of the bending assembly, which determines the minimum achievable bend radius and the spatial extent of the deformation zone along the longitudinal axis of the orthopedic implant at each discrete segment; a maximum achievable deformation angle per bend operation, which represents the largest angular deformation that the bending assembly can apply in a single bending cycle without exceeding the mechanical limits of the actuation subsystem or the force magnifier mechanism; a feeding resolution specification defining the minimum linear displacement increment of the feeding assembly (i.e., the smallest corresponding segment length that the feeding assembly can reliably advance) and the minimum rotational increment of the rotator motor (i.e., the smallest angular change in target deformation orientation that the feeding assembly can reliably execute); and an actuator characteristic specification defining the maximum force output, maximum velocity, acceleration profile, and positional accuracy of the servo motor and the stepper motors of the implant fabrication device 160.

The planning module 150 utilises the device capability profile to constrain and optimise the numerical decomposition of the desired rod profile into the finite sequence of discrete bending operations. For example, if the maximum achievable deformation angle per bend operation of the implant fabrication device 160 is limited to a predetermined threshold (e.g., 20 degrees), the planning module 150's generated instruction set is limited in that no individual target deformation angle in the deformation target data set exceeds this threshold, and if the desired rod profile requires a localised angular change that exceeds the threshold, the planning module 150 distributes the required angular change across two or more adjacent discrete segments having shorter corresponding segment lengths such that each individual target deformation angle remains within the device's operational limits. Similarly, the planning module 150 ensures that no corresponding segment length in the deformation target data set is smaller than the minimum linear displacement increment of the feeding assembly, and that no target deformation orientation change between successive discrete segments is smaller than the minimum rotational increment of the rotator motor.

In some embodiments, the planning module 150 further adapts the implant fabrication instruction set 161 to the physical characteristics of the orthopedic implant itself, including the implant material (e.g., cobalt-chromium or titanium alloy), the implant diameter (e.g., 5.5 mm or 6.0 mm), and the implant cross-sectional geometry (e.g., circular or non-circular). Different implant materials exhibit different yield strengths, elastic moduli, and strain-hardening behaviours, which affect both the force and dwell time required to achieve a given target deformation angle and the magnitude of elastic spring-back after the bending force is removed. The planning module 150 may access a material properties database storing, for each supported implant material and diameter combination, empirically determined or analytically computed parameters including the elastic modulus, the yield stress, the ultimate tensile strength, the strain-hardening exponent, and a spring-back compensation lookup table or parametric model that maps target deformation angles to expected spring-back magnitudes.

The planning module 150 incorporates the implant-material-specific parameters into the deformation target data set by computing, for each discrete segment, a recommended initial overbend angle and a recommended predetermined dwell period that are specific to the implant material and diameter, thereby enabling the controller circuit of the implant fabrication device 160 to achieve the target deformation angle with fewer iterative correction cycles. In some embodiments, the material properties database is updated based on data from past fabrications of implants of comparable material, diameter, and accumulated bend history, such that the spring-back compensation parameters are refined over time as additional fabrication data is collected.

In some embodiments, the planning module 150 generates the implant fabrication instruction set 161 in a format that encodes not only the deformation target data set but also device-specific operational commands, including motor velocity profiles for each discrete segment (e.g., specifying a rapid approach velocity, a deceleration profile upon contact detection based on the force measurement system 2, a controlled deformation velocity during the bending phase, and a retraction velocity after the predetermined dwell period), force thresholds for contact detection (e.g., specifying the bending force measurement value from the force measurement system 2 at which the controller circuit transitions from the rapid approach phase to the controlled deformation phase), and convergence criteria for the iterative spring-back compensation algorithm (e.g., specifying the predetermined tolerance for the angle measurement system and the maximum number of iterative correction cycles before the controller circuit flags the discrete segment for operator review).

If a spine stiffness parameter has been received from the stiffness measuring device 120, the planning module 150 further modifies the implant fabrication instruction set 161 by applying an overbend correction to the deformation target data set, such that when the orthopedic implant is attached and the spine exerts its resistive forces on the orthopedic implant, the person 101's spine assumes the desired postoperative alignment.

In some embodiments, the planning module 150 renders a fabrication preview via the user interface 180, the fabrication preview comprising a graphical animation or sequential visualisation of the discrete bending operations as would be executed by the implant fabrication device 160, enabling the operator to verify the planned fabrication sequence before committing to physical deformation of the orthopedic implant.

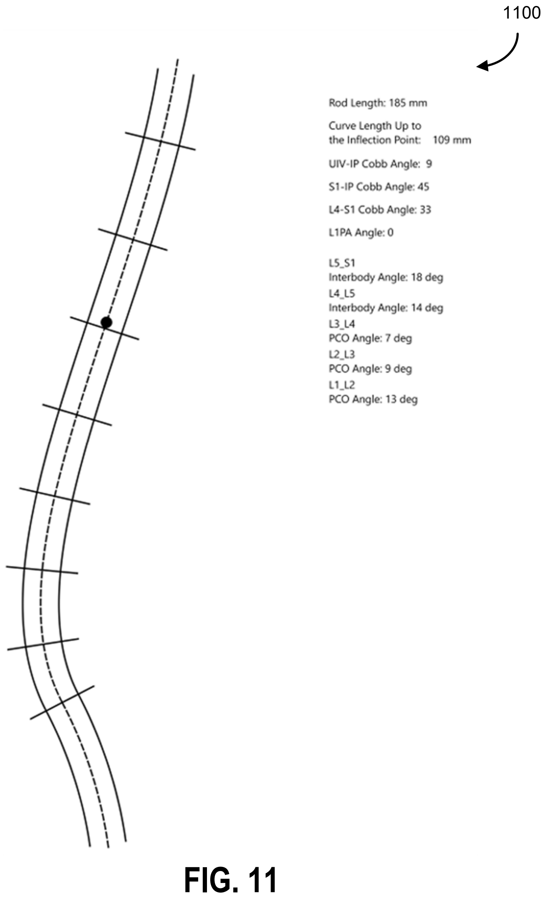

**FIG. 11** illustrates a rod profile output 1100 generated by the planning module 150. While the implant fabrication instruction set 161 is a machine-readable data object, the planning module 150 may render the fabrication instruction set 161 for display at the user interface 180 or as a printable visual reference for manual verification of the fabricated implant in the operating room. The rod profile output 1100 shows the rod length (e.g., 185 mm), the curve length up to the inflection point (e.g., 109 mm), the UIV-to-inflection-point Cobb angle (e.g., 9 degrees), the S1-to-inflection-point Cobb angle (e.g., 45 degrees), the L4-S1 Cobb angle (e.g., 33 degrees), the L1PA angle, and per-level interbody angles and PCO angles for each vertebral level in the fused region.

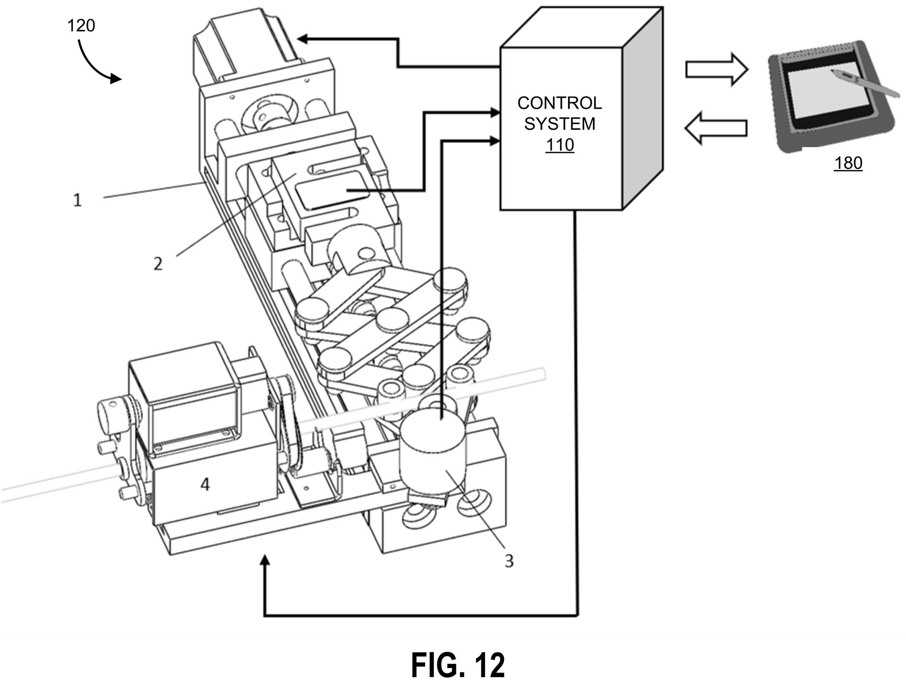

Subsequently, the implant fabrication device 160 receives and executes the implant fabrication instruction set 161 generated by the planning module 150 to fabricate the implant. **FIG. 12** is a schematic diagram showing the integration of the control system 110, the user interface 180, and the hardware subsystems of the implant fabrication device 160. The implant fabrication device 160 includes the following hardware subsystems: at least one actuation subsystem 1 comprising a bending mechanism driven by at least one actuator, such as an electromotor, a hydraulic actuator, or a pneumatic actuator, a plurality of linkages magnifying a bending force applied by the bending mechanism, and a plurality of rollers providing three-point bending for a cylindrical metal implant 16; at least one force measurement system 2 comprising at least one sensor and an appropriate analog-to-digital converter to interface with the control system 110; at least one angle measurement system 3 comprising at least one sensor and an appropriate interface that logs a bending angle of the cylindrical metal implant 16; and a feeding system 4 capable of receiving commands from the control system 110 to grip, linearly advance, and rotationally orient the cylindrical metal implant 16 within the bending mechanism. The control system 110 receives data from the force measurement system 2 and the angle measurement system 3, and provides commands to the actuation subsystem 1 and the feeding system 4.

In the force measurement system 2, at least one sensor is used to report a bending force measurement value to the control system 110. The sensor can be a load cell that converts a mechanical load into an analog electrical voltage. The analog electrical voltage is then converted by an analog-to-digital converter and transmitted to a microcontroller of the control system 110. The bending force measurement is forwarded to the user interface 180 for graphical rendering and is referenced by the control system 110 in the actuation of the bending mechanism of the actuation subsystem 1. The force measurement system 2 may measure one or more of a bending force, a shear force, and a normal force.

In the angle measurement system 3, at least one sensor is used to report a bending angle of the cylindrical metal implant 16 to the control system 110. The sensor of the angle measurement system 3 can be contact-based or non-contact-based, for example an optical encoder or a magnetic encoder. The encoder outputs a series of pulses that represents the angular displacement of the encoder shaft. In an alternative embodiment, the sensor output does not directly represent the deformation angle of the cylindrical metal implant. In this case, the control system 110 computes the deformation angle based on the encoder output (e.g., a displacement measurement or a position measurement) and a set of known geometric relationships between the encoder shaft position and the roller contact geometry.

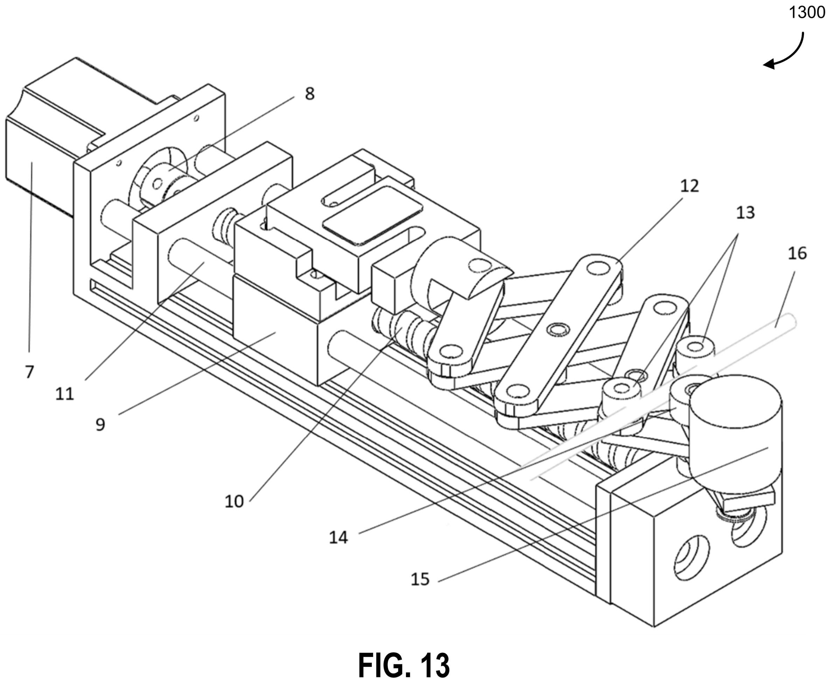

**FIG. 13** illustrates an isometric view 1300 of an example implant fabrication device 160. The implant fabrication device 160 comprises the bending assembly and the feeding assembly together with their associated drive mechanisms. In the depicted embodiment, the actuation subsystem 1 is driven by a servo motor 7. A servo drive circuit receives either a position command or a velocity command from the control system 110 and energises the servo motor 7 accordingly. The servo motor 7 is mechanically coupled to a ball screw 10 via a coupling 8, or alternatively via a timing belt and pulley arrangement. The ball screw 10 converts the rotational motion of the servo motor 7 into linear motion of a saddle 9 that translates along an axis perpendicular to the bending axis of the cylindrical metal implant 16. The translation axis of the saddle 9 is supported by linear guides 11 and ball bearings to ensure smooth, low-friction motion. A rod support 15 holds the cylindrical metal implant 16 in position within the bending region. The bending assembly collectively encompasses the actuation subsystem 1, including the servo motor 7, coupling 8, saddle 9, ball screw 10, guides 11, force magnifier mechanism 12, and the bending rollers 13 and 14.

To reduce the required motor torque and the overall physical size of the servo motor 7 , a force magnifier mechanism 12 is disposed between the saddle 9 and the bending rollers. The force magnifier mechanism 12 comprises a plurality of linkages that utilise mechanical advantage to magnify the bending force applied by the bending mechanism at the expense of displacement. Specifically, the saddle 9's displacement is greater than the roller displacement, therefore the resulting force at the rollers 13 and 14 is mechanically magnified as compared to the force at the saddle 9. This linkage arrangement enables the use of a smaller, lower-power servo motor 7 while still delivering sufficient bending force to plastically deform the cylindrical metal implant 16, which may be fabricated from high-strength alloys such as CoCr or titanium.

The bending assembly effects a controlled deformation of the orthopedic implant via a three-point bending mechanism comprising a plurality of rollers. In a symmetrical bending configuration, there are two moving rollers 13 and one fixed roller 14. The two moving rollers 13 rotate symmetrically around the fixed roller 14 while applying a bending force to the cylindrical metal implant 16 disposed between them, thereby bending the cylindrical metal implant 16 to a target deformation angle at the point of contact with the fixed roller 14. In an alternative asymmetrical bending configuration, there are two fixed rollers and one moving roller. The single moving roller rotates around one of the fixed rollers, thereby bending the cylindrical metal implant 16 between the rollers. Whether symmetrical or asymmetrical, the three-point bending mechanism provides a precision bend to a specific target deformation angle at each discrete segment. Once a first bend at a first discrete segment is completed, the bending mechanism retracts, the feeding assembly advances the cylindrical metal implant 16 by the corresponding segment length of the next discrete segment, and the bending mechanism applies the next controlled deformation, such that the totality of the controlled deformations across all discrete segments yields a final orthopedic implant whose cumulative bend profile maps to the desired rod profile specified in the deformation target data set.

The controller circuit within the control system 110 is configured to apply a spring-back compensation magnitude when applying each controlled deformation. The spring-back compensation magnitude is the additional angular deformation applied beyond the target deformation angle to account for elastic recovery of the cylindrical metal implant 16 material. The elastic deformation properties of the orthopedic implant are a function of the implant material (e.g., CoCr versus titanium), the implant diameter, and the accumulated strain history from prior bending operations. To compensate for this elastic spring-back, the controller circuit implements the following iterative control algorithm: the control system 110 receives the target deformation angle for the current discrete segment from the deformation target data set. The servo motor 7 of the actuation subsystem 1 is then commanded to advance the bending mechanism rapidly toward the cylindrical metal implant 16. The control system 110 continuously monitors the bending force measurement from the force measurement system 2 while the bending mechanism is in motion. At the onset of contact between the rollers 13, 14 and the cylindrical metal implant 16, the bending force measurement increases rapidly, and the servo motor 7 is commanded by the control system 110 to decelerate as the controlled deformation commences. The bending assembly advances to deform the orthopedic implant beyond the target deformation angle (e.g., 2 degrees beyond the target deformation angle) and holds the deformed position for a predetermined dwell period before the servo motor 7 is commanded to retract. The servo motor 7 is then commanded to retract slowly until the bending force measurement from the force measurement system 2 drops to zero.

At this point, the controller circuit receives an intermediate state comprising a resulting deformation angle of the cylindrical metal implant 16 as measured by the angle measurement system 3. If the resulting deformation angle is less than the target deformation angle (i.e., elastic spring-back has caused the implant to recover past the target), the controller circuit repeats the deforming and retracting sequence. The control system 110 iterates this sequence until the measured deformation angle matches the target deformation angle within a predetermined tolerance. The predetermined tolerance is a maximum permissible angular difference (e.g., ±0.5 degrees) between the measured resulting deformation angle and the target deformation angle. The magnitude of the overbending and the duration of the predetermined dwell period may be adjusted by the control system 110 based on data from a past fabrication of an implant of comparable material, diameter, and accumulated bend history.

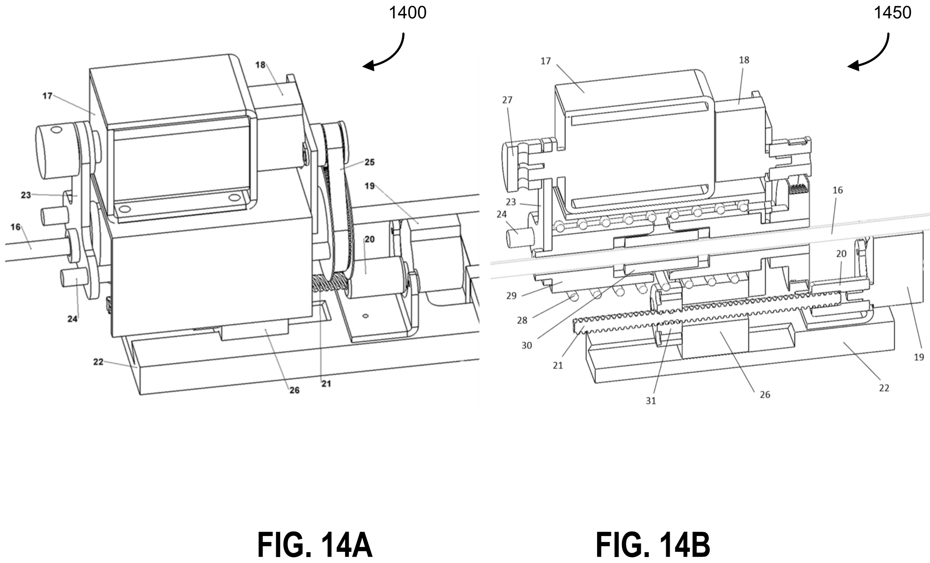

**FIG. 14A** illustrates a close-up isometric view 1400 of a first embodiment of the feeding assembly, and **FIG. 14B** illustrates a cross-section 1450 of the same feeding assembly. The feeding assembly of **FIG. 14A** and **FIG. 14B** is configured to frictionally engage, linearly advance, and rotationally orient the cylindrical metal implant 16 within the bending mechanism. In the depicted embodiment, the feeding assembly includes at least one quick-response actuator, such as an electro-magnetic solenoid 17, that provides a frictional grip action when energised by the control system 110. The grip action of the solenoid 17 is activated when the controller circuit commands the feeding assembly to advance a discrete segment of the cylindrical metal implant 16 into the bending region of the bending assembly. When the required corresponding segment length of the cylindrical metal implant 16 has been advanced into the bending zone, the control system 110 de-energises the solenoid 17, thereby releasing the grip action. The feeding assembly is then retracted to its original position by a stepper motor 19 in preparation for the next advancing motion.

Before the controlled deformation of each discrete segment is commenced, the feeding assembly is commanded by the control system 110 to grip the cylindrical metal implant 16 and advance it by the corresponding segment length toward the bending mechanism. The corresponding segment length is determined by the control system 110 based on the mathematical decomposition of the desired rod profile into discrete segments, as specified in the deformation target data set, to ensure that the resulting bend is smooth along the finished orthopedic implant.

In some embodiments of the feeding assembly, there are two V-shaped grippers 30 located on opposite sides (e.g., top and bottom) of the cylindrical metal implant 16. The V-shaped grippers 30 provide frictional gripping action as they are driven toward the cylindrical metal implant 16 by two wedge components 29 when the solenoid 17 is energised. In operation, the solenoid 17 pushes the two wedge components 29 in a direction parallel to the longitudinal axis of the cylindrical metal implant 16 , and the inclined surfaces of the wedge components 29 translate this axial force into a perpendicular clamping force that drives the V-shaped grippers 30 inward toward the cylindrical metal implant 16 , thereby contacting the cylindrical metal implant 16 and providing frictional engagement through normal force. The V-shaped grippers 30 are spring-loaded to ensure that when the solenoid 17 is not energised, the V-shaped grippers 30 retract and no contact exists between the feeding assembly and the cylindrical metal implant 16. In some embodiments, the solenoid 17 transmits the axial pushing force via a feed cap 23 that mechanically connects to the wedge components 29. The feed cap 23 is a removable component that facilitates disassembly for sterilisation. The feeding assembly further comprises a housing that includes a rod guide channel 24, guide structures 22, springs 26, and a spring retainer 28 that cooperate with the wedge components 29 and V-shaped grippers 30 to maintain proper alignment of the cylindrical metal implant 16 during advancing and rotation operations. A solenoid cap 27 retains the solenoid 17 within the housing and is removable to facilitate the sterilisation procedure. An additional housing element 31 provides structural support to the feeding assembly.

The feeding assembly is further configured to adjust a bending orientation of the orthopedic implant according to the target deformation orientation specified in the deformation target data set. In some embodiments, the wedge components 29 and the V-shaped grippers 30 can be rotated around the longitudinal axis of the cylindrical metal implant 16 (while the grip action is active) by a stepper motor 18 to a specific rotational angle commanded by the control system 110. The stepper motor 18 drives one of the wedge components 29 via a timing belt and pulley 25, causing the entire gripper assembly to rotate about the implant axis. This rotation alters the rotational orientation of the cylindrical metal implant 16 such that subsequent controlled deformations by the bending assembly are applied on different planes. For example, if the cylindrical metal implant 16 is rotated by 180 degrees at an inflection point in the desired rod profile by the stepper motor 18, the direction of the resulting bend reverses, producing an S-shaped implant whose curvature changes direction at the inflection point. The cylindrical metal implant 16 may be rotated to any arbitrary angle between 0 and 360 degrees to enable bending on multiple planes, thereby enabling the fabrication of orthopedic implants with complex three-dimensional curvatures as required for patients with combined sagittal and coronal deformity, such as scoliosis.

In some embodiments, the feeding assembly, including the wedge components 29, the V-shaped grippers 30, and the solenoid 17 actuator, translates linearly along the longitudinal axis of the cylindrical metal implant 16. This linear translation is driven by the stepper motor 18 connected to a screw system 21 via a coupling 20. The linear translation distance is commanded by the control system 110 and executed by the stepper motor 18. The feeding assembly thus functions as a linearly movable clamp that is intermittently activated (via the solenoid 17) to frictionally advance successive discrete segments of the orthopedic implant into the bending assembly.

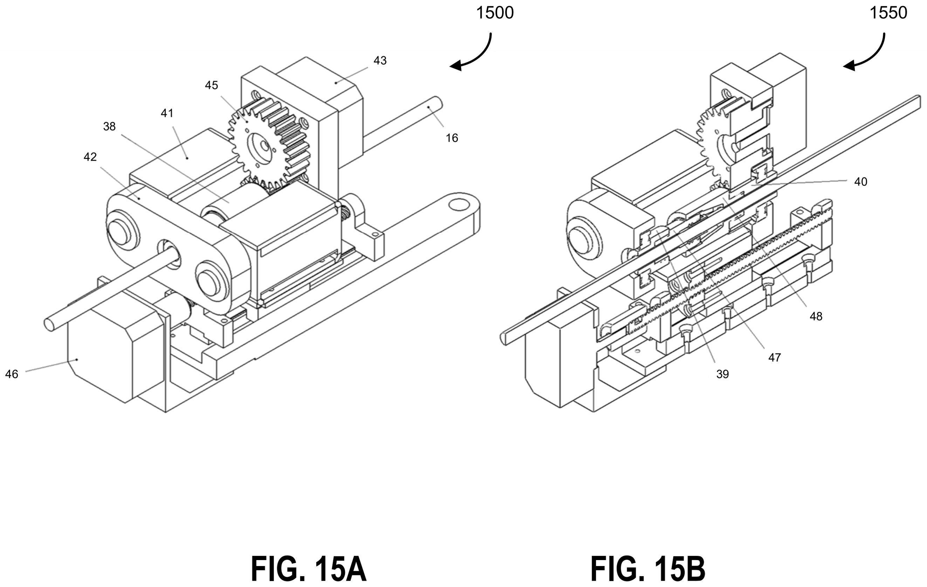

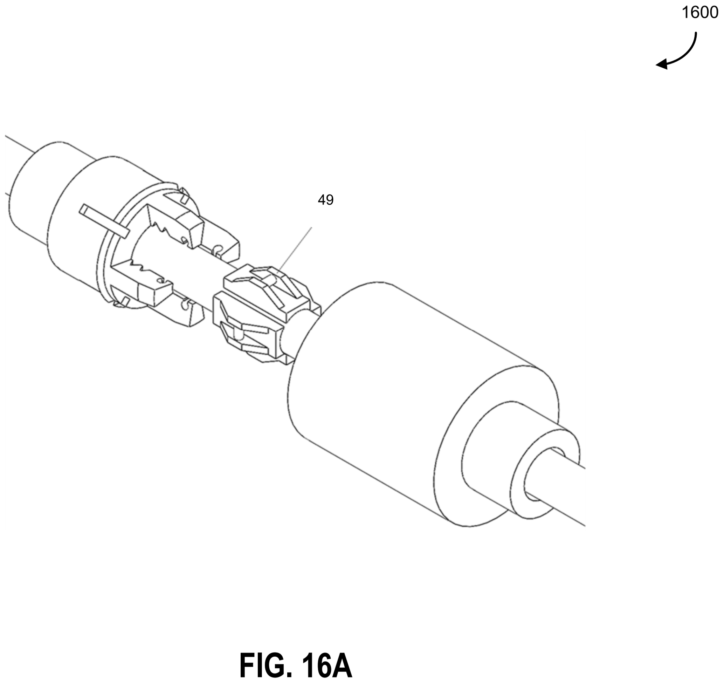

**FIG. 15A** illustrates a close-up isometric view 1500 of an alternative embodiment of the feeding assembly, and **FIG. 15B** illustrates a cross-section 1550 of the same alternative feeding assembly. **FIG. 16A** illustrates a close-up isometric view 1600 of the gripper elements of the alternative feeding assembly that are obscured in **FIG. 15A****.** In this alternative embodiment, a disposable sterilised cartridge 38 is employed as the frictional gripper element to advance and rotate the cylindrical metal implant 16. The sterilised cartridge 38 is screwed in and fixed to a pusher 39 and a support 40 that are supported by ball bearings for smooth rotational and axial motion. Two actuators 41 pull the pusher 39 and the support 40 together via a cap part 42, generating the clamping force. A first motor 43 spins the clamped cylindrical metal implant 16 via a set of gears 45, providing the rotational adjustment of the bending orientation. A second motor 46 translates the feeding assembly longitudinally along the axis of the cylindrical metal implant 16, providing the linear advance corresponding to each corresponding segment length in the deformation target data set.

Referring to **FIG. 16A****,** when the sterilised cartridge 38 is pushed axially by the actuators 41 via the cap part 42, a cartridge support 47 and a cartridge pusher 48 move toward each other, forcing four grippers 49 to converge inward and grasp the cylindrical metal implant 16. The grippers 49 are hinged around a protrusion of the pusher 39, providing mechanical rigidity and self-alignment with the longitudinal axis of the cylindrical metal implant 16. The four-gripper arrangement of the grippers 49 provides a symmetrical clamping force distribution around the circumference of the cylindrical metal implant 16, reducing the risk of surface marking or deformation at the grip point. The sterilised cartridge 38 is a disposable component that is discarded after each surgical procedure and replaced with a new sterilised cartridge 38 before the next procedure.

The feeding assembly, the sensor subsystems (including the force measurement system 2 and the angle measurement system 3), and the bending contact surfaces of the actuation subsystem 1 (including the rollers 13 and 14) are designed to be disassembled by hand such that components that contact the cylindrical metal implant 16 or the patient can be easily sterilised for use in a surgical setting. In the embodiment of **FIG. 14A** and **FIG. 14B****,** a method for removing the contact components from the feeding assembly for sterilisation comprises removing the solenoid cap 27, followed by placing a magnet bar inside the feeding assembly housing, energising and de-energising the solenoid 17, and then removing the feed cap 23. Energising and de-energising the solenoid 17 causes the V-shaped grippers 30 and the wedge components 29 to adhere to the magnet bar, thus enabling the components to be withdrawn when the magnet bar is removed. The same sequence is followed in reverse to reassemble the sterilised components into the feeding assembly. In the alternative embodiment of **FIG. 15A** and **FIG. 15B**, the rod gripper subassembly is disassembled by inserting a tool rod into the grippers 49, unscrewing a nut behind the support 40, and withdrawing the subassembly. The sterilised cartridge 38 is then removed and discarded, and the remaining components are sterilised and reassembled with a new sterilised cartridge 38. The rest of the system components that do not contact the cylindrical metal implant 16 may be physically covered and do not require frequent sterilisation. The ease of sterilisation of the contact components aids the deployment of the implant fabrication device 160 in intraoperative use cases, wherein the implant fabrication device 160 is situated and operated in the operating room for real-time fabrication and iterative correction of the orthopedic implant during the surgical procedure.

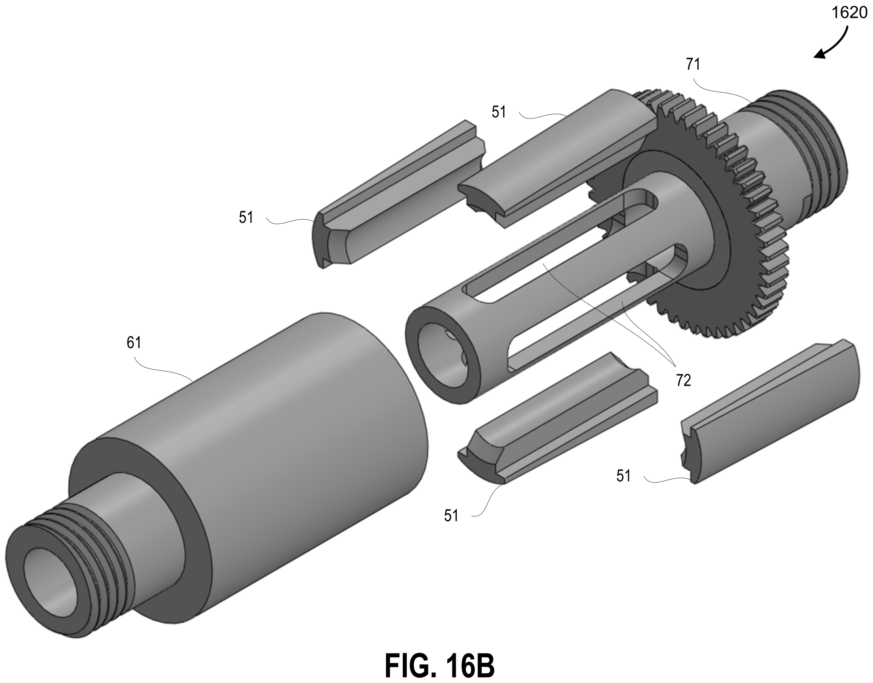

**FIGS. 16B-16E** illustrate alternative embodiments of the gripper assembly for use within the feeding assembly of the implant fabrication device 160. The alternative gripper assembly embodiments as shown in **FIGS. 16B-16E** may be employed in place of the four grippers 49 of **FIG. 16A** within the alternative feeding assembly of **FIG. 15A**, or may be adapted for use with other feeding assembly configurations. In the alternative gripper assembly of **FIGS. 16B-16E****,** gripper jaws 51 translate longitudinal actuation by biasing element 71 into clamping force against a cylindrical implant rod. Each gripper jaw 51 has a wedge-shaped profile such that when the gripper jaws 51 are received within an outer sleeve 61, the internal surface 62 guides the gripper jaws 51 between open and closed configurations around a cylindrical implant rod upon a longitudinal actuation by the biasing element 71.

**FIG. 16B** illustrates an isometric view 1620 of an alternative embodiment of the gripper assembly in a disassembled configuration. The gripper assembly comprises a plurality of gripper jaws 51 arranged symmetrically about a longitudinal axis. The gripper jaws 51 are configured to converge radially inward to frictionally engage the cylindrical metal implant when the feeding assembly is actuated. The alternative gripper assembly of **FIG. 16B** further comprises an outer sleeve 61 within which the gripper jaws 51 are slidably received, and a biasing element 71 having a slot 72, the biasing element 71 leaving the gripper jaws 51 in an open configuration when the feeding assembly is not actuated. The biasing element 71 may comprise a spring, an elastomeric element, or another resilient member that stores mechanical energy when the gripper jaws 51 are driven inward and releases the stored energy to return the gripper jaws 51 to the open configuration upon removal of the actuating force. The slot 72 of the biasing element 71 receives a corresponding portion of the gripper jaw 51, axially distributing the gripper jaws 51 around the rod implant. The dimensions of the slot 72 may be configured such that the gripper jaw 51 is constrained to move only in one dimension upon actuation, either towards or away from the longitudinal axis. Such an arrangement reduces the risk of unintentionally loose engagement that could cause misalignment of the cylindrical metal implant during feeding and rotation operations.

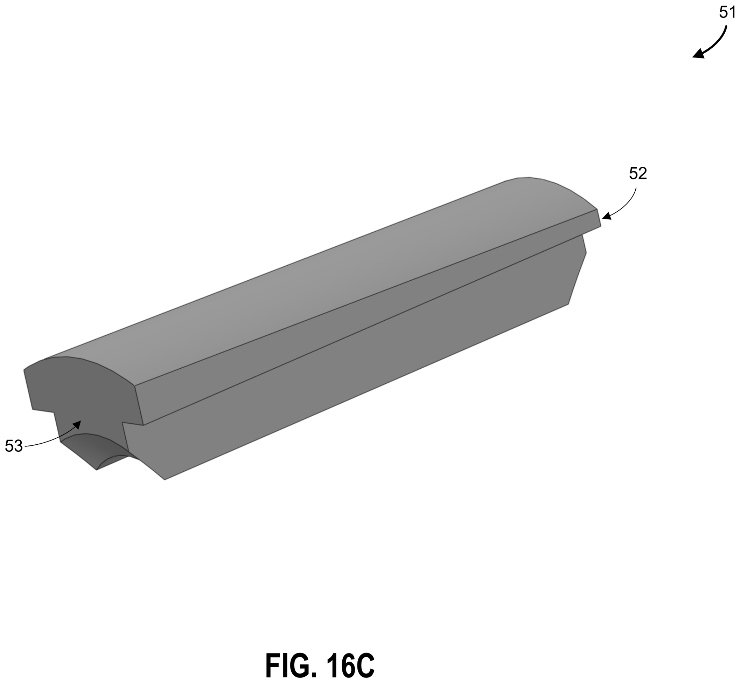

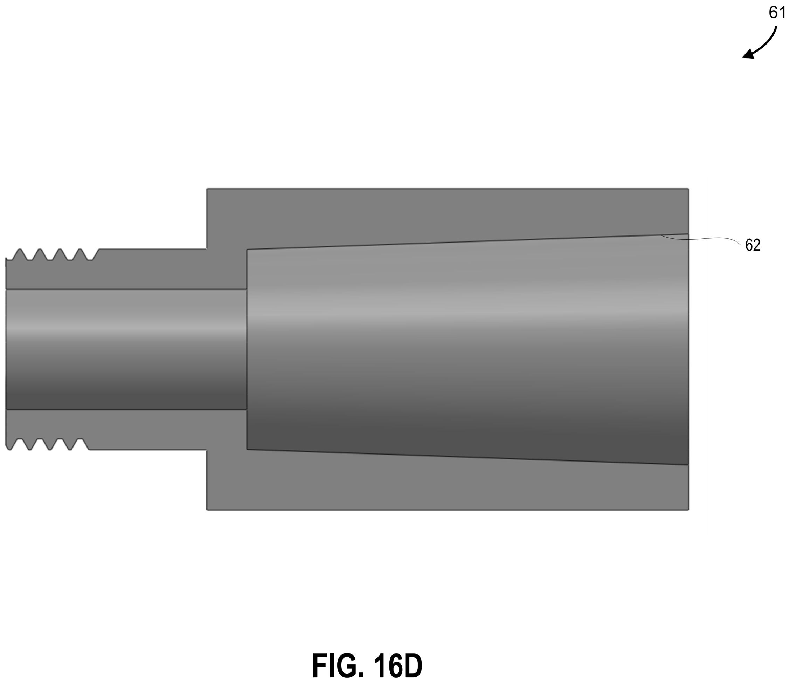

**FIG. 16C** illustrates a detail view of an individual gripper jaw 51 of the alternative gripper assembly of **FIG. 16B****.** Each gripper jaw 51 comprises a gripper jaw body having a wedge-shaped profile that tapers from a thicker end 52 to a thinner end 53. The wedge-shaped profile of the gripper jaw 51 cooperates with the internal surface 62 of the outer sleeve 61 (shown in **FIG. 16D**) such that axial displacement of the gripper jaws 51 relative to the outer sleeve 61 causes the gripper jaws 51 to converge radially inward toward the closed configuration, gripping the rod implant, or to retract radially outward toward the open configuration, releasing the rod implant. In some embodiments, the inner surface of gripper jaw 51 is contoured to conform to the curvature of the cylindrical metal implant, for example as a concave arcuate surface having a radius of curvature that matches or slightly exceeds the outer radius of the cylindrical metal implant, thereby maximizing the contact area between the gripper jaw 51 and the cylindrical metal implant. The resulting distribution of clamping force over a larger surface area also reduces the risk of surface marking or localized deformation at the gripping point. In some embodiments, the gripper jaw 51 is fabricated from a material that is compatible with sterilisation procedures, such as surgical-grade stainless steel or a medical-grade polymer, and the bottom surface of gripper jaw 51 may be textured, knurled, or coated with a high-friction material to enhance the frictional engagement with the cylindrical metal implant.

**FIG. 16D** illustrates a detail view of the outer sleeve 61 of the alternative gripper assembly of **FIG. 16B****.** The outer sleeve 61 is a structural component of the gripper assembly that provides a cylindrical housing within which the gripper jaws 51 are slidably received. The outer sleeve 61 defines an internal surface 62 that is shaped to cooperate with the wedge-shaped profile of the gripper jaws 51. As the gripper jaws 51 are displaced axially within the outer sleeve 61, the tapered geometry of the gripper jaws 51 (tapering from the thicker end 53 to the thinner end 52) causes the internal surface 62 of the outer sleeve 61 to cam the gripper jaws 51 radially inward toward the closed configuration or to permit the gripper jaws 51 to retract radially outward toward the open configuration. The outer sleeve 61 and the internal surface 62 together provide a self-centring collet-like mechanism that ensures the gripper jaws 51 converge concentrically toward the longitudinal axis of the cylindrical metal implant when actuated, maintaining coaxial alignment between the cylindrical metal implant and the feeding assembly during advancing and rotation operations in cooperation with other subassemblies.

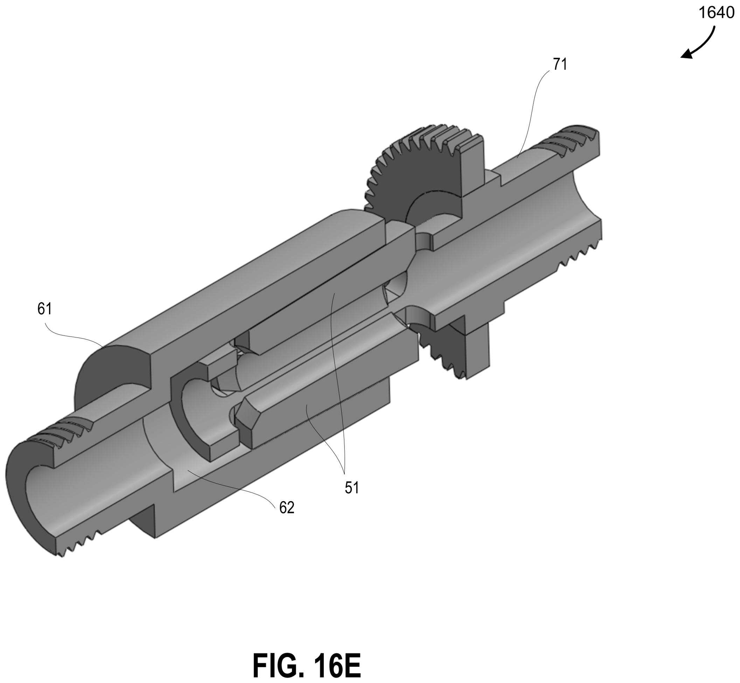

**FIG. 16E** illustrates a cutaway view 1640 of the alternative gripper assembly of **FIG. 16B** in an assembled state, showing the gripper jaws 51 received within the outer sleeve 61, with the biasing element 71 in position. In the assembled state of **FIG. 16E**, the gripper jaws 51 are slidably received within the outer sleeve 61 and are urged toward the open configuration by the biasing element 71. When the actuators 41 of the feeding assembly apply an axial force via the cap part 42, the gripper jaws 51 are driven axially within the outer sleeve 61 such that the wedge-shaped profile of the gripper jaws 51 cooperates with the internal surface 62 to converge radially inward and clamp the cylindrical metal implant. Upon removal of the axial force, the biasing element 71 returns the gripper jaws 51 to the open configuration, releasing the cylindrical metal implant. The alternative gripper assembly of **FIGS. 16B-16E** provides a modular gripper configuration that can be readily separated into its constituent components for sterilisation between surgical procedures, and reassembled by reinserting the gripper jaws 51 into the outer sleeve 61 and reattaching the biasing element 71.

In some embodiments, the feeding of the metal implant and the clamping of the metal implant may be accomplished by the same axial actuation of the biasing element 71. The axial force moves the gripper jaws 51 into the closed configuration, and subsequent actuation of the biasing element 71 is thus translated directly to the gripped metal implant. Embodiments of the biasing element 71 may additionally be rotationally actuated by a rotator motor, such that when gripper jaws 51 are in the closed configuration, the biasing element can simultaneously provide movement in both axial and rotational modes, serving multiple functions within the same assembly.

Referring back to **FIGs 1-2**, the intraoperative verification module 170 is configured to receive, as input, intraoperative spinal alignment data obtained after attachment of the orthopedic implant to the person 101's spine. The intraoperative spinal alignment data may be acquired by intraoperative imaging (e.g., intraoperative X-ray, fluoroscopy, or EOS imaging) and subsequent processing by the measurement module 130 and simulation module 140 as previously described. The intraoperative verification module 170 compares the intraoperative spinal alignment data against the desired postoperative spinal alignment on a segment-by-segment basis. The intraoperative verification module 170 further recomputes the estimated musculoskeletal forces at each vertebral level based on the intraoperative spinal alignment data and determines whether the forces at the UIV and UIV+1 levels exceed predetermined thresholds. The output of the intraoperative verification module 170 is either: (a) a verification of compliance, indicating that the intraoperative spinal alignment data is within a predetermined tolerance of the desired postoperative spinal alignment across each of the instrumented vertebral levels; or (b) a non-compliance determination, in which case the intraoperative verification module 170 triggers the planning module 150 to generate an updated implant fabrication instruction set 161 for execution by the implant fabrication device 160, enabling the orthopedic implant to be removed, re-processed, and reattached before surgical closure. The intraoperative verification module 170 renders the segment-by-segment deviations as color-coded indicators (e.g., red, green, yellow) on the user interface 180, enabling rapid visual assessment of which vertebral levels are within tolerance and which require correction.

The user interface 180 provides the graphical display through which the operator interacts with the system 100 across all stages of the computational pipeline. The user interface 180 receives rendering data from the measurement module 130, the simulation module 140, the planning module 150, and the intraoperative verification module 170, and renders graphical representations for display, including: graphical spine representations and vertebral-level visualisations from the measurement module 130; parameter tables, simulation displays, cone of economy visualisations, force comparison displays, and risk factor computation outputs from the simulation module 140; color-coded sagittal and coronal deviation displays and per-level adjustment controls from the planning module 150; rod profile outputs and fabrication status from the planning module 150 and the implant fabrication device 160; and compliance or non-compliance indicators from the intraoperative verification module 170. The user interface 180 further receives operator input specifying the UIV and LIV selection, the desired alignment classification criterion, corrective procedures at individual vertebral levels, and manual adjustments to the desired rod profile. The user interface 180 may be implemented as an electronic device such as a personal computer, a mobile phone, a tablet computer, or a stand-alone monitor or panel. The communication pathway between the user interface 180 and the control system 110 may be wired (e.g., USB, Ethernet) or wireless (e.g., Wi-Fi, Bluetooth).

In some embodiments, the user interface 180 is further configured to receive operator input via a voice-based interaction modality. The user interface 180 comprises a microphone or microphone array communicatively coupled to a speech recognition engine executed by the computer processor of the control system 110. The speech recognition engine converts spoken operator commands into structured input events that are interpreted by the measurement module 130, the simulation module 140, the planning module 150, or the intraoperative verification module 170 in the same manner as input events received via keyboard, mouse, or touch input. For example, the operator may issue a spoken command to select a candidate realignment configuration (e.g., "select GAP alignment"), to specify a corrective procedure at a designated vertebral level (e.g., "apply PCO at L4-L5"), to initiate fabrication (e.g., "begin rod fabrication"), or to request a force analysis (e.g., "run musculoskeletal analysis"). The voice-based interaction modality is advantageous in the intraoperative setting because the operating surgeon's hands may be occupied with surgical instruments or within the sterile field, and voice commands enable the surgeon to interact with the control system 110 and the implant fabrication device 160 without requiring physical contact with a keyboard, mouse, or touchscreen.

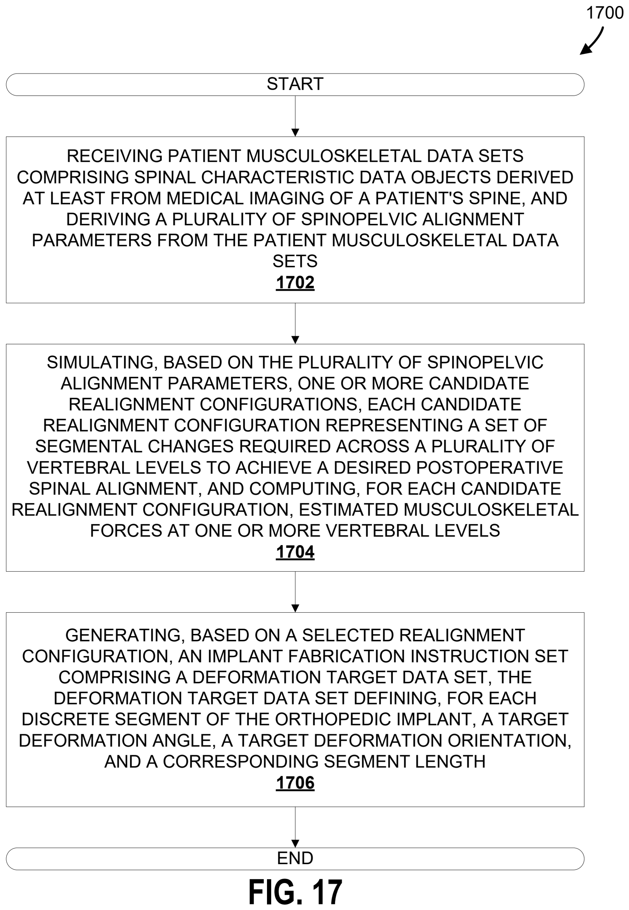

**FIG. 17** illustrates a flowchart 1700 of the computational steps of a method for automated intraoperative formation of an orthopedic implant for attachment during spinal fusion surgery. The flowchart 1700 begins with a step 1702 of receiving patient musculoskeletal data sets comprising spinal characteristic data objects derived at least from medical imaging of a patient's spine, and deriving a plurality of spinopelvic alignment parameters from the patient musculoskeletal data sets. The flowchart 1700 proceeds to a step 1704 of simulating, based on the plurality of spinopelvic alignment parameters, one or more candidate realignment configurations, each candidate realignment configuration representing a set of segmental changes required across a plurality of vertebral levels to achieve a desired postoperative spinal alignment, and computing, for each candidate realignment configuration, estimated musculoskeletal forces at one or more vertebral levels. The flowchart 1700 then proceeds to a step 1706 of generating, based on a selected realignment configuration, an implant fabrication instruction set 161 comprising a deformation target data set, the deformation target data set defining, for each discrete segment of the orthopedic implant, a target deformation angle, a target deformation orientation, and a corresponding segment length. The steps 1702, 1704, and 1706 of the flowchart 1700 are executed by the computer processor of the control system 110 and correspond to the operations of the measurement module 130, the simulation module 140, and the planning module 150, respectively.

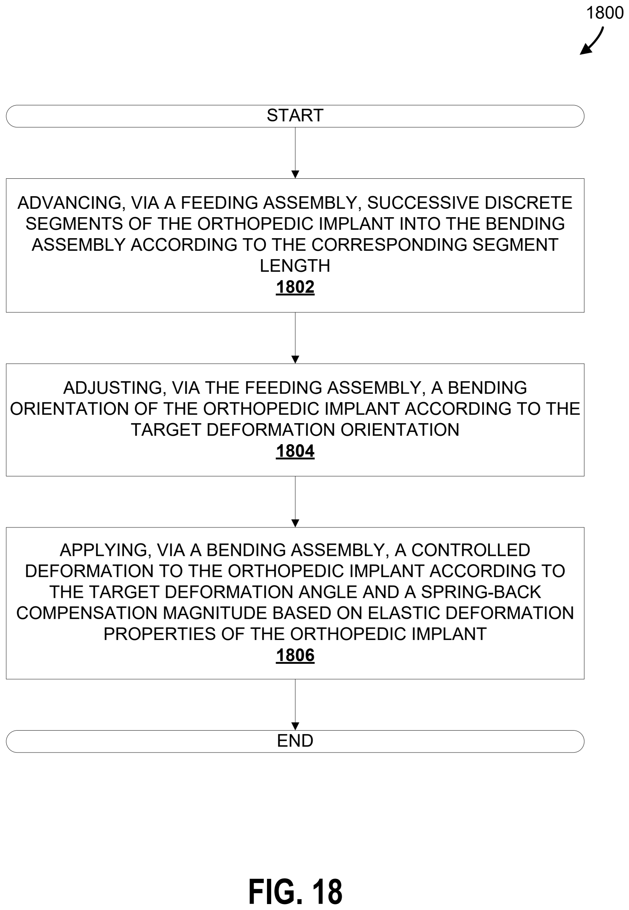

**FIG. 18** illustrates a flowchart 1800 of the execution steps of the implant fabrication instruction set 161 at the implant fabrication device 160. The flowchart 1800 begins with a step 1802 of advancing, via the feeding assembly, successive discrete segments of the orthopedic implant into the bending assembly according to the corresponding segment length. The flowchart 1800 proceeds to a step 1804 of adjusting, via the feeding assembly, a bending orientation of the orthopedic implant according to the target deformation orientation. The flowchart 1800 then proceeds to a step 1806 of applying, via the bending assembly, a controlled deformation to the orthopedic implant according to the target deformation angle and a spring-back compensation magnitude based on elastic deformation properties of the orthopedic implant. The steps 1802, 1804, and 1806 of the flowchart 1800 are repeated for each discrete segment of the orthopedic implant as specified in the deformation target data set, such that the complete sequence of controlled deformations yields a finished orthopedic implant conforming to the selected candidate realignment configuration.

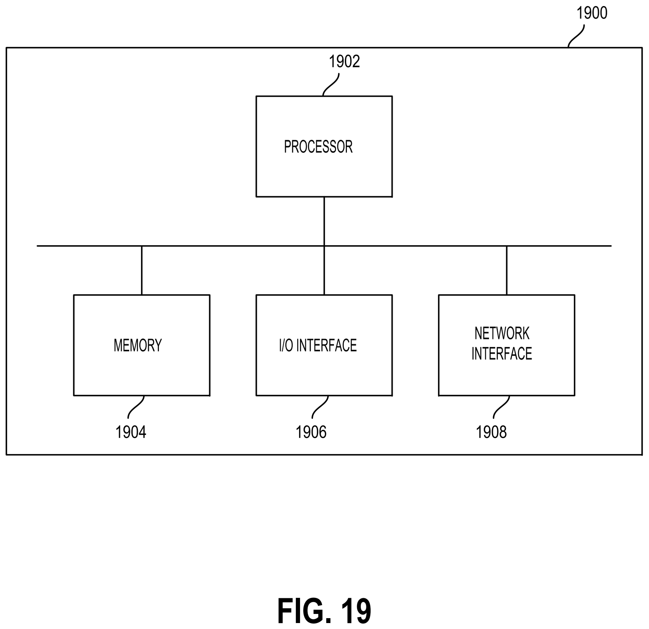

**FIG. 19** is an example computing device 1900, according to various embodiments. The computing device 1900, such as a computer server, can be utilised to implement the control system 110, the measurement module 130, the simulation module 140, the planning module 150, or the intraoperative verification module 170. The computing device 1900 includes one or more processors 1902, which operate in conjunction with computer memory 1904.

The computer memory 1904 can be adapted to store various non-transitory computer readable instruction sets, which can be executed on the processors 1902 to cause the performance of various methods described herein. Such instruction sets may implement the measurement module 130 to receive patient musculoskeletal data sets comprising spinal characteristic data objects and derive a plurality of spinopelvic alignment parameters therefrom, invoke the simulation module 140 to simulate one or more candidate realignment configurations and compute estimated musculoskeletal forces at one or more vertebral levels, execute the planning module 150 to generate an implant fabrication instruction set 161 comprising a deformation target data, and run the intraoperative verification module 170 to compare intraoperative spinal alignment data against the desired postoperative spinal alignment and verify fabrication compliance.

The computer memory 1904 can maintain data objects representing the patient musculoskeletal data sets, the spinal characteristic data objects, the plurality of spinopelvic alignment parameters, the candidate realignment configurations with corresponding simulated postoperative spinal geometries, the estimated musculoskeletal forces at each vertebral level, the risk factor of mechanical complication, the spine stiffness parameter, the deformation target data set, and the implant fabrication instruction set 161. An input/output interface 1908 can be utilised to receive various input commands, for example, through an attached keyboard, mouse, or touch input, and generate various outputs, such as screen renderings for display on a computer display of the user interface 180. The computer memory 1904 can store both the raw spinal characteristic data objects derived from medical imaging and the derived spinopelvic alignment parameters with their corresponding alignment classification metadata for retrieval during simulation and planning. The input/output interface 1908 may expose operator controls for specifying the upper instrumented vertebra and lower instrumented vertebra selection, selecting a candidate realignment configuration from among the plurality of alignment classification criteria, applying corrective procedures at individual vertebral levels via per-level adjustment controls, and inspecting the force comparison display 900 and risk factor computation output 1000 associated with a selected candidate realignment configuration.

A networking interface 1906 can be utilised to communicate one or more data sets, for example, through a wired connection (e.g., USB, Ethernet) or a wireless connection (e.g., Wi-Fi, Bluetooth). The networking interface 1906 can be adapted to receive patient musculoskeletal data sets from external medical imaging systems, for example, as the measurement and simulation process takes place.

The computing device 1900, in some embodiments, can be implemented as a special-purpose machine deployed in an operating room or integrated into a surgical cart. The special-purpose machine is a purpose-built embedded computing platform that consolidates the computational pipeline and the electromechanical control functions of the system 100 into a single sterilizable-environment-compatible unit that is configured for point-of-care operation during a surgical procedure. The special-purpose machine may take the form of a compact rack-mounted or cart-mounted industrial computer comprising a sealed or fan-filtered enclosure rated for the particulate and humidity conditions of an operating theatre, a medical-grade power supply, and a touchscreen or externally connected display serving as the user interface 180. The special-purpose machine comprises a controller CPU connected to (i) dedicated memory storing the material properties database with empirically determined spring-back compensation parameters for each supported implant material and diameter combination, (ii) dedicated processing units - which may include one or more graphics processing units (GPUs), field-programmable gate arrays (FPGAs), or application-specific integrated circuits (ASICs) - for executing the trained artificial intelligence model for unfused spine prediction and the patient-specific musculoskeletal model for estimated musculoskeletal force computation, (iii) a real-time motor control interface for transmitting position commands and velocity commands to the servo motor 7 and stepper motors 18, 19 of the implant fabrication device 160 and receiving sensor feedback from the force measurement system 2 and the angle measurement system 3, (iv) a graphical rendering engine for generating the colour-coded spine visualisations, the three-dimensional spine reconstruction, the cone of economy visualisation, the force comparison display 900 , the risk factor computation output 1000 , and the rod profile output 1100 for display on the user interface 180 , and (v) a communication interface for receiving spine stiffness parameter data from the stiffness measuring device 120 and intraoperative spinal alignment data from intraoperative imaging systems.

The special-purpose machine interfaces with the implant fabrication device 160 to transmit the implant fabrication instruction set 161 comprising the deformation target data set for autonomous execution by the controller circuit. Remote devices, including medical imaging systems such as biplanar radiographic systems (e.g., EOS), computed tomography scanners, magnetic resonance imaging scanners, and inertial measurement units positioned on the patient's vertebrae, communicate with the computing device 1900 via the networking interface 1906 to provide patient musculoskeletal data sets for ingestion by the measurement module 130. In some embodiments, the special-purpose machine further comprises a local data storage subsystem for archiving patient musculoskeletal data sets, implant fabrication instruction sets, and fabrication log data (including per-segment bending force measurements, angle measurements, and spring-back compensation iterations) for each surgical procedure, enabling retrospective analysis, quality assurance, and refinement of the material properties database over successive procedures. Within the computational pipeline, the measurement module 130, the simulation module 140, and the planning module 150 process the patient musculoskeletal data sets sequentially to produce the implant fabrication instruction set 161, and the intraoperative verification module 170 receives post-attachment alignment data to drive the closed-loop iterative correction workflow. Downstream subsystems receive the implant fabrication instruction set 161 via the input/output interface 1908 to drive autonomous implant fabrication, intraoperative verification, and iterative rod correction.

In a non-limiting practical implementation example, biplanar radiographic images (e.g., EOS imaging) and computed tomography (CT) imaging are acquired of a person 101 presenting with degenerative lumbar scoliosis and sagittal imbalance. The spinal characteristic data objects derived from the medical imaging further comprise dynamic motion data sets from one or more inertial measurement units (IMUs) positioned on the person 101's vertebrae preoperatively, the dynamic motion data sets being representative of patient-specific dynamic sagittal alignment and acquired while the person 101 performs functional activities such as sit-to-stand transitions. The computer processor of the control system 110 receives the imaging data and the dynamic motion data sets as patient musculoskeletal data sets comprising the spinal characteristic data objects derived from the medical imaging of the person 101's spine. Where CT imaging is available, the spinal characteristic data objects further encode volumetric bone density information from which a bone mineral density of the person 101 is derived. At step 1702 of the method of **FIG. 17****,** the measurement module 130 automatically derives a plurality of spinopelvic alignment parameters from the patient musculoskeletal data sets, including pelvic incidence, sacral slope, pelvic tilt, lumbar lordosis (L1-S1 and L4-S1), thoracic kyphosis (T4-T12), sagittal vertical axis, T1 pelvic angle, global tilt, GAP score, and segmental Cobb angles at each vertebral level, as rendered in **FIG. 4****.**

At step 1704, the simulation module 140 simulates one or more candidate realignment configurations for a planned fusion from T10 to the sacrum (UIV = T10, LIV = S1), generating the GAP alignment 502, Segmental alignment 504, Age-adjusted alignment 506, T4-L1-axis alignment 508, and Roussouly alignment 510 as shown in **FIG. 5****.** For each candidate realignment configuration, the simulation module 140 computes estimated musculoskeletal forces at one or more vertebral levels from T12 to S1, including shear forces and compression forces at each intervertebral level and at the UIV (T10) and UIV+1 (T11) levels, by solving a patient-specific musculoskeletal model that receives as input the patient's sex, weight, height, and simulated postoperative spinal curvature. The computer processor integrates the dynamic motion data sets into the musculoskeletal force computation such that the estimated musculoskeletal forces at each vertebral level reflect both the static standing alignment and the dynamic loading patterns expected during the person 101's habitual activities, as rendered in the force comparison of **FIG. 9****.** The computer processor further computes a risk factor of mechanical complication for each candidate realignment configuration, the risk factor computed based on the estimated musculoskeletal forces (including the dynamic forces) at one or more vertebral levels in combination with the bone mineral density of the person 101 derived from the CT imaging, as rendered in the risk factor computation output of **FIG. 10****.** The risk factor of mechanical complication comprises an estimated probability in the occurrence of one or more of proximal junctional failure, proximal junctional kyphosis, rod fracture, sacral fracture, adjacent segment disease, and pseudoarthrosis. The planning module 150 identifies candidate realignment configurations that minimise peak dynamic forces at the UIV and UIV+1 levels, thereby reducing the risk factor of mechanical complication that may be underestimated by static force analysis alone. The planning module 150 further modifies the implant fabrication instruction set 161 to minimise the risk factor of mechanical complication.

The planning module 150 compares the one or more candidate realignment configurations against the plurality of alignment classification criteria and renders the cone of economy visualisation as shown in **FIG. 6****,** enabling the operator to determine that the GAP cone display 620 places the patient's centre of gravity most centrally within the cone of economy. The user interface 180 renders the sagittal simulation 700 and coronal simulation 800 of **FIGs. 7-8****,** with colored bars at each vertebral level indicating the segmental Cobb angle deviation from the desired postoperative spinal alignment. The operator specifies corrective procedures (e.g., PCO at L4-L5, interbody cage insertion at L5-S1) using the per-level adjustment controls, and the color-coded bars update in real time.

Upon selection of the GAP-aligned candidate realignment configuration, the spine of the person 101 is surgically modified through osteotomy and release procedures (e.g., posterior column osteotomy at L4-S1 and pedicle subtraction osteotomy at L3). The stiffness measuring device 120 then applies a predetermined displacement or force at one or more vertebral levels of the surgically modified spine and measures the resulting force or displacement to derive a spine stiffness parameter at each measured level. The computer processor of the control system 110 receives the spine stiffness parameter. At step 1706 of **FIG. 17****,** the planning module 150 generates the implant fabrication instruction set 161 comprising the deformation target data set based on the rod profile output 1100 of **FIG. 11****,** which defines for each discrete segment of the orthopedic implant the target deformation angle, target deformation orientation, and corresponding segment length. The planning module 150 modifies the implant fabrication instruction set 161 by applying an overbend correction to the deformation target data set based on the spine stiffness parameter, such that the target deformation angles are increased by amounts computed from the spine stiffness parameter to account for the spine's resistive forces. The user interface 180 renders the stiffness-corrected rod profile alongside the original target rod profile.

At steps 1802, 1804, and 1806 of the flowchart 1800 of **FIG. 18****,** the implant fabrication device 160 executes the implant fabrication instruction set 161: the controller circuit causes the feeding assembly to advance successive discrete segments of the orthopedic implant into the bending assembly according to each corresponding segment length, adjust the bending orientation of the orthopedic implant according to each target deformation orientation by commanding the stepper motor 18 to rotate the cylindrical metal implant 16 to the appropriate angle, and apply, via the bending assembly, a controlled deformation to the orthopedic implant according to each target deformation angle and a spring-back compensation magnitude based on elastic deformation properties of the orthopedic implant. For each discrete segment, the controller circuit applies the spring-back compensation magnitude by advancing the bending assembly to deform the orthopedic implant beyond the target deformation angle, holding the deformed position for the predetermined dwell period, retracting the bending assembly, measuring a resulting deformation angle of the orthopedic implant after retraction via the angle measurement system 3, and repeating the deforming and retracting until the measured deformation angle matches the target deformation angle within the predetermined tolerance. The implant fabrication device 160 thereby compensates for both elastic spring-back of the orthopedic implant and, through the overbend correction, the anticipated resistive forces of the spine.

The fabricated orthopedic implant is attached to pedicle screws at the instrumented vertebral levels from T10 to S1. When the orthopedic implant is attached, the spine's resistive stiffness acts upon the orthopedic implant, and the overbend correction ensures that the resulting equilibrium geometry of the person 101's spine assumes the desired postoperative alignment. Intraoperative spinal alignment data is then obtained by acquiring intraoperative imaging (e.g., intraoperative EOS or fluoroscopic imaging) after attachment of the orthopedic implant. The intraoperative verification module 170 of the control system 110 receives the intraoperative spinal alignment data, compares the intraoperative spinal alignment data against the desired postoperative spinal alignment on a segment-by-segment basis, and renders the deviations as color-coded indicators on the user interface 180. The intraoperative verification module 170 further recomputes the estimated musculoskeletal forces at each vertebral level based on the measured intraoperative spinal alignment and determines whether the segmental Cobb angle deviation at one or more vertebral levels exceeds the predetermined tolerance, or whether the estimated musculoskeletal forces at the UIV+1 level exceed a threshold value indicative of elevated risk of proximal junctional kyphosis.

If the intraoperative verification module 170 determines that the intraoperative spinal alignment data does not comply with the desired postoperative spinal alignment, the intraoperative verification module 170 triggers the planning module 150 to generate an updated implant fabrication instruction set 161 for execution by the implant fabrication device 160, the updated implant fabrication instruction set 161 comprising an updated deformation target data set that corrects for the measured segment-by-segment deviations. The orthopedic implant is removed from the pedicle screws and re-processed by the implant fabrication device 160 according to the updated implant fabrication instruction set 161. The intraoperative verification module 170 then verifies compliance to the desired postoperative spinal alignment before surgical closure proceeds. The entire measurement, simulation, fabrication, verification, and iterative correction cycle is completed intraoperatively.

Applicant notes that the described embodiments and examples are illustrative and non-limiting. Practical implementation of the features may incorporate a combination of some or all of the aspects, and features described herein should not be taken as indications of future or existing product plans.

The term "connected" or "coupled to" may include both direct coupling (in which two elements that are coupled to each other contact each other) and indirect coupling (in which at least one additional element is located between the two elements).

Although the embodiments have been described in detail, changes, substitutions and alterations can be made herein without departing from the scope. Moreover, the scope of the present application is not intended to be limited to the particular embodiments of the process, machine, manufacture, composition of matter, means, methods and steps described in the specification.

Processes, machines, manufacture, compositions of matter, means, methods, or steps, presently existing or later to be developed, that perform substantially the same function or achieve substantially the same result as the corresponding embodiments described herein may be utilized. Accordingly, the embodiments disclosed herein are intended to include within their scope such processes, machines, manufacture, compositions of matter, means, methods, or steps.

As can be understood, the examples described above and illustrated are intended to be exemplary only.

## Claims

1. A system for automated intraoperative formation of an orthopedic implant for attachment during spinal fusion surgery, the system comprising:
a computer processor coupled with computer memory and a non-transitory computer-readable storage medium, the computer processor configured to:
receive patient musculoskeletal data sets comprising spinal characteristic data objects derived at least from medical imaging of a patient's spine, and derive a plurality of spinopelvic alignment parameters from the patient musculoskeletal data sets;
simulate, based on the plurality of spinopelvic alignment parameters, one or more candidate realignment configurations, each candidate realignment configuration representing a set of segmental changes required across a plurality of vertebral levels to achieve a desired postoperative spinal alignment, and compute, for each candidate realignment configuration, estimated musculoskeletal forces at one or more vertebral levels; and
generate, based on a selected realignment configuration, an implant fabrication instruction set comprising a deformation target data set, the deformation target data set defining, for each discrete segment of the orthopedic implant, a target deformation angle, a target deformation orientation, and a corresponding segment length;
an implant fabrication device comprising a bending assembly, a feeding assembly, and a controller circuit, the controller circuit configured to cause the implant fabrication device to:
advance, via the feeding assembly, successive discrete segments of the orthopedic implant into the bending assembly according to the corresponding segment length;
adjust, via the feeding assembly, a bending orientation of the orthopedic implant according to the target deformation orientation; and
apply, via the bending assembly, a controlled deformation to the orthopedic implant according to the target deformation angle and a spring-back compensation magnitude based on elastic deformation properties of the orthopedic implant.

2. The system of claim 1, wherein the computer processor is further configured to:
receive intraoperative spinal alignment data obtained after attachment of the orthopedic implant;
compare the intraoperative spinal alignment data against the desired postoperative spinal alignment; and
verify a compliance to the desired postoperative spinal alignment or generate an updated implant fabrication instruction set for execution by the implant fabrication device.

3. The system of claim 1, further comprising a stiffness measurement subsystem configured to apply a predetermined displacement or force onto the patient's spine and measure a resulting force or displacement to derive a spine stiffness parameter; wherein the computer processor is further configured to receive the spine stiffness parameter and to modify the implant fabrication instruction set by applying an overbend correction to the deformation target data set, such that the patient's spine assumes the desired postoperative alignment when the orthopedic implant is attached.

4. The system of claim 1, wherein the computer processor is further configured to modify the implant fabrication instruction set to minimize a risk factor of mechanical complication, the risk factor of mechanical complication computed based on the estimated musculoskeletal forces at one or more vertebral levels in combination with a bone mineral density of the patient.

5. The system of claim 4, wherein the risk factor of mechanical complication comprises an estimated probability in the occurrence of one or more of proximal junctional failure, proximal junctional kyphosis, rod fracture, sacral fracture, adjacent segment disease, and pseudoarthrosis.

6. The system of claim 1, wherein the computer processor is further configured to render a graphical display of the plurality of spinopelvic alignment parameters of a simulated baseline spine and the desired postoperative spinal alignment as colored bars, each colored bar indicating a degree of deviation from the desired postoperative spinal alignment.

7. The system of claim 6, wherein the computer processor is further configured to render for display in real time as the baseline spine is modified toward the desired postoperative spinal alignment, segmental Cobb angles at each vertebral level in one or both of sagittal and coronal planes, wherein each vertebra is rendered with a color corresponding to a magnitude of difference between a baseline Cobb angle and a target Cobb angle.

8. The system of claim 1, wherein the computer processor is further configured to compare the one or more candidate realignment configurations against a plurality of alignment classification criteria comprising one or more of: a Global Alignment and Proportion classification, an age-adjusted alignment, a Roussouly classification, and a segmental alignment, the computer processor further configured to render a cone of economy visualisation indicating musculoskeletal expenditure for each candidate realignment configuration.

9. The system of claim 1, wherein the spinal characteristic data objects further comprise dynamic motion data sets from one or more inertial measurement units positioned on the patient's vertebrae, the dynamic motion data sets representative of patient-specific dynamic sagittal alignment.

10. The system of claim 1, wherein the controller circuit is configured to apply the spring-back compensation magnitude by: advancing the bending assembly to deform the orthopedic implant beyond the target deformation angle; holding the deformed position for a predetermined dwell period; retracting the bending assembly; measuring a resulting deformation angle of the orthopedic implant after retraction; and repeating the deforming and retracting until the measured deformation angle matches the target deformation angle within a predetermined tolerance.

11. A method for automated intraoperative formation of an orthopedic implant for attachment during spinal fusion surgery, the method comprising:
receiving patient musculoskeletal data sets comprising spinal characteristic data objects derived at least from medical imaging of a patient's spine, and deriving a plurality of spinopelvic alignment parameters from the patient musculoskeletal data sets;
simulating, based on the plurality of spinopelvic alignment parameters, one or more candidate realignment configurations, each candidate realignment configuration representing a set of segmental changes required across a plurality of vertebral levels to achieve a desired postoperative spinal alignment, and computing, for each candidate realignment configuration, estimated musculoskeletal forces at one or more vertebral levels;
generating, based on a selected realignment configuration, an implant fabrication instruction set comprising a deformation target data set, the deformation target data set defining, for each discrete segment of the orthopedic implant, a target deformation angle, a target deformation orientation, and a corresponding segment length;
executing the implant fabrication instruction set at an implant fabrication device by:
advancing, via a feeding assembly, successive discrete segments of the orthopedic implant into the bending assembly according to the corresponding segment length;
adjusting, via the feeding assembly, a bending orientation of the orthopedic implant according to the target deformation orientation;
applying, via a bending assembly, a controlled deformation to the orthopedic implant according to the target deformation angle and a spring-back compensation magnitude based on elastic deformation properties of the orthopedic implant.

12. The method of claim 11, further comprising:
receiving intraoperative spinal alignment data obtained after attachment of the orthopedic implant;
comparing the intraoperative spinal alignment data against the desired postoperative spinal alignment; and
verifying a compliance to the desired postoperative spinal alignment or generating an updated implant fabrication instruction set for execution by the implant fabrication device.

13. The method of claim 11, further comprising applying a predetermined displacement or force onto the patient's spine and measuring a resulting force or displacement to derive a spine stiffness parameter; and modifying the implant fabrication instruction set by applying an overbend correction to the deformation target data set, such that the patient's spine assumes the desired postoperative alignment when the orthopedic implant is attached.

14. The method of claim 11, further comprising modifying the implant fabrication instruction set to minimize a risk factor of mechanical complication, the risk factor of mechanical complication computed based on the estimated musculoskeletal forces at one or more vertebral levels in combination with a bone mineral density of the patient.

15. A non-transitory computer readable medium storing computer interpretable instruction sets, which when executed by a computer processor, cause the computer processor to perform the method of any one of claims 11-14.
